# EUROPÄISCHE PATENTSCHRIFT

(11) **EP 1 027 399 B1**
(45) Veröffentlichungstag und Bekanntmachung des Hinweises auf die Patenterteilung: **02.02.2005**
(21) Anmeldenummer: 98949997.5
(22) Anmeldetag: 08.09.1998
(51) Int. Cl.: C09K 11/06, H05B 33/14

(54) **TRIPTYCENDERIVATE UND IHRE VERWENDUNG FÜR OPTOELEKTRONISCHE ANWENDUNGEN, INSBESONDERE ALS ELEKTROLUMINESZENZMATERIALIEN**
TRYPTICENE DERIVATIVES AND THEIR USE IN OPTOELECTRONIC DEVICES, IN PARTICULAR AS ELECTROLUMINESCENT MATERIALS
DERIVES DE TRIPTYCENE ET LEUR UTILISATION DANS DES DISPOSITIFS OPTOELECTRONIQUES, EN PARTICULIER COMME MATERIAUX ELECTROLUMINESCENTS

(30) Priorität: 10.10.1997 DE 19744792
(43) Veröffentlichungstag der Anmeldung: 16.08.2000
(73) Patentinhaber: Covion Organic Semiconductors GmbH, 65926 Frankfurt (DE)
(72) Erfinder: SALBECK, Josef, D-65779 Kelkheim (DE); BECKER, Heinrich, D-61479 Glashütten (DE); KREUDER, Willi, D-55126 Mainz (DE); WEINFURTNER, Karl, Heinz, 93057 Regensburg (DE)
(74) Vertreter: Luderschmidt, Schüler & Partner GbR
(86) Internationale Anmeldenummer: PCT/EP1998/005686
(87) Internationale Veröffentlichungsnummer: WO 1999/019419

(56) Entgegenhaltungen:
- JP-A- 5 015 809
- US-A- 4 539 507
- WASIELEWSKI, MICHAEL R. ET AL: "High-quantum-yield long-lived charge separation in a photosynthetic reaction center model" J. AM. CHEM. SOC. (1985), 107(19), 5562-3 CODEN: JACSAT;ISSN: 0002-7863, XP002092033
- WASIELEWSKI, MICHAEL R. ET AL: "Ultrafast photoinduced electron transfer in rigid donor-spacer-acceptor molecules: modification of spacer energetics as a probe for superexchang" TETRAHEDRON (1989), 45(15), 4785-806 CODEN: TETRAB;ISSN: 0040-4020, XP002092034

## Beschreibung

Es besteht ein hoher industrieller Bedarf an großflächigen Festkörper-Lichtquellen für eine Reihe von Anwendungen, überwiegend im Bereich von Anzeigeelementen, der Bildschirmtechnologie und der Beleuchtungstechnik. Die an diese Lichtquellen gestellten Anforderungen können zur Zeit von keiner der bestehenden Technologien völlig befriedigend gelöst werden.

Als Alternative zu herkömmlichen Anzeigeelementen, wie Glühlampen, Gasentladungslampen und nicht selbstleuchtenden Flüssigkristallanzeigeelementen, sind bereits seit einiger Zeit Elektrolumineszenz(EL)materialien und -vorrichtungen, wie lichtemittierende Dioden (LED), bekannt.

Elektrolumineszenzmaterialien sind Stoffe, die befähigt sind, beim Anlegen eines elektrischen Feldes Licht abzustrahlen. Das physikalische Modell zur Beschreibung dieses Effektes basiert auf der strahlenden Rekombination von Elektronen und Elektronenlücken ("Löchern"). Bei lichtemittierenden Dioden werden die Ladungsträger über die Kathode bzw. Anode in das Elektrolumineszenzmaterial injiziert.
Elektrolumineszenzvorrichtungen enthalten ein Lumineszenzmaterial als lichtemitterende Schicht.
Allgemein sind Elektrolumineszenzmaterialien und -vorrichtungen beispielsweise beschrieben in Ullmann's Encyclopedia of Industrial Chemistry, Vol A9, 5th Ed. VCH Verlag 1987 und der dort zitierten Literatur.
Neben anorganischen Stoffen, wie ZnS/Mn oder GaAs, sind auch organische Verbindungen als EL-Materialien bekannt geworden.

Eine Beschreibung von EL-Vorrichtungen, die niedermolekulare organischen EL-Materialien enthalten, findet sich beispielsweise in US 4,539,507.

Obwohl mit solchen Materialien gute Ergebnisse erzielt wurden, bietet das Eigenschaftsprofil solcher Verbindungen noch durchaus Raum für Verbesserungen.

Da zudem die Entwicklung von Elektrolumineszenzmaterialien noch in keiner Weise als abgeschlossen betratet werden kann, sind die Hersteller von Beleuchtungs- und Anzeigevorrichtungen nach wie vor an den unterschiedlichsten Elektrolumineszenzmaterialien für solche Vorrichtungen interessiert.

Dies liegt unter anderem auch daran, daß erst das Zusammenwirken des Elektrolumineszenzmaterials mit den weiteren Bauteilen der Vorrichtungen Rückschlüsse auf die Eignung auch des Elektrolumineszenzmaterials zuläßt.

Aufgabe der vorliegenden Erfindung war es daher, neue Elektrolumineszenzmaterialien bereitzustellen, die bei Verwendung in Beleuchtungs- oder Anzeigevorrichtungen geeignet sind, das Eigenschaftsprofil dieser Vorrichtungen zu verbessern.

Es wurde nun überraschend gefunden, daß sich bestimmte Derivate des Triptycens in besonderer Weise zur Verwendung als Elektrolumineszenzmaterialien eignen.

Ein Gegenstand der Erfindung ist daher die Verwendung eines Triptycenderivats der Formel (I) in einer Elektrolumineszenzvorrichtung, 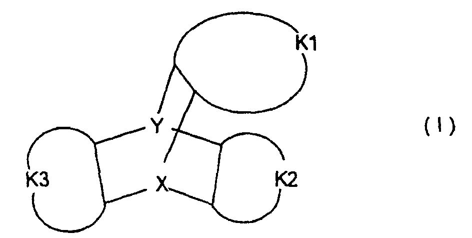 wobei die Symbole in der Formel folgende Bedeutungen haben:
- K¹,K²,K³: sind, gleich oder verschieden, mono- oder polycyclische Systeme, die gegebenenfalls Heteroatome, vorzugsweise N, S, O, enthalten und die gegebenenfalls substituiert sind;
- X, Y: sind, gleich oder verschieden, CR¹, N, P, As, SiR²;
- R¹: ist, gleich oder verschieden, H, Halogen, Pseudohalogen oder ein Kohlenwasserstoffrest mit 1 bis 30 C-Atomen, der gegebenenfalls auch Heteroatome, vorzugsweise -O-, -N- und/oder -S-, enthält;
- R²: ist, gleich oder verschieden, ein Kohlenwasserstoffrest mit 1 bis 30 C-Atomen, der gegebenenfalls auch Heteroatome, vorzugsweise -O-, -N- und /oder -S-, enthält.

Verbindungen der Formel (I) zeichnen sich durch eine ausreichende bis gute Löslichkeit in gängigen organischen Lösungsmitteln, gute Filmbildungseigenschaften und eine verringerte Tendenz zur Kristallisation aus. Dadurch wird die Herstellung von Elektrolumineszenzvorrichtungen erleichtert und ihre Lebensdauer erhöht. Die Emissionseigenschaften der erfindungsgemäß eingesetzten Verbindungen können durch die Wahl geeigneter Substituenten über den ganzen Bereich des sichtbaren Spektrums eingestellt werden. Darüber hinaus erlaubt die kovalent gebundene Anordnung der verschiedene Teile der Triptycenverbindung einen molekularen Aufbau in der Weise, daß in verschiedenen Teilen des Moleküls unabhängig bestimmte Eigenschaften eingestellt werden können. So kann ein Teil z.B. Ladungstransport- oder Ladungsinjektionseigenschaften besitzen, während der andere lichtemittierende Eigenschaften besitzt.

Vorzugsweise ist mindestens einer der Systeme K¹⁻³ ein Fluorophor. Ein Fluorophor im Sinne der Erfindung ist eine Atom-Gruppierung, die dem Triptycenderivat Fluoreszenz verleiht, beispielsweise ein ausgedehntes' aromatisches System.

Weiterhin bevorzugt ist es, daß alle drei Systeme K¹⁻³ konjugiert sind.

Bevorzugte, gegebenenfalls substituierte und/oder bi- oder polycyclische konjugierte Systeme sind: 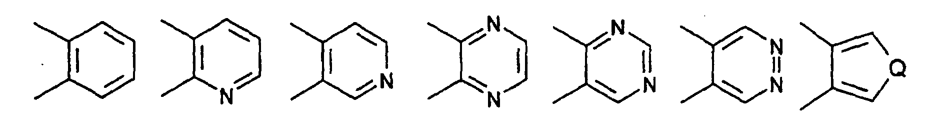 mit Q = S, O, NR².

Besonders bevorzugte Verbindungen der allgemeinen Formel (I) sind Triptycenderivate der Formel (II) und Formel (III), 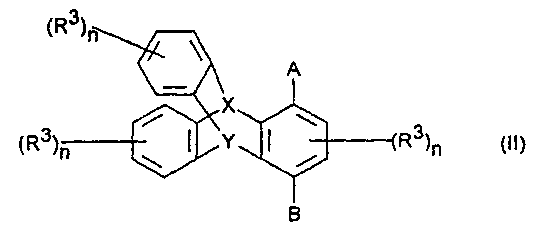 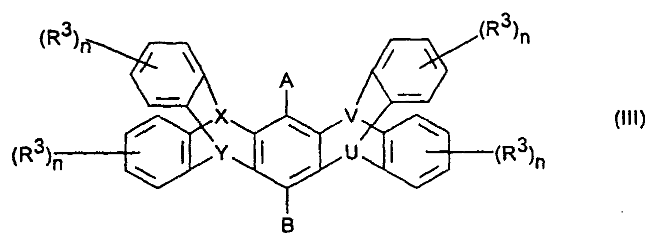 wobei die Symbole und Indizes folgende Bedeutungen haben:
- X, Y, U, V: sind, gleich oder verschieden, CR¹, N, P, As, SiR²;
- R¹: ist, gleich oder verschieden, H, Halogen, Pseudohalogen oder ein Kohlenwasserstoffrest mit 1 bis 30 C-Atomen, der gegebenenfalls auch Heteroatome, vorzugsweise -O-, -N- und/oder -S-, enthalten kann;
- R²: ist, gleich oder verschieden, ein Kohlenwasserstoffrest mit 1 bis 30 C-Atomen, der gegebenenfalls auch Heteroatome, vorzugsweise -O-, -N-und/oder -S-, enthalten kann.
- R³: ist, gleich oder verschieden, F, Cl, Br, I, CN, NO₂, eine unverzweigte oder verzweigte Alkylgruppe mit 1 bis 22 C-Atomen, wobei eine oder mehrere -CH₂-Gruppen durch -O-, -S-, -SO₃-, -O-CO-, -CO-O-, Aryl oder Heteroaryl (mit jeweils 4 bis 10 C-Atomen) ersetzt sein können, mit der Maßgabe, daß nicht zwei Sauerstoffatome unmittelbar miteinander gebunden sein dürfen, und wobei ein, mehrere oder alle H-Atome durch F ersetzt sein können, und wobei zwei am selben Ring befindliche Substituenten R² unter Ausbildung eines Ringes oder weiteren anellierten Ringsystems miteinander verknüpft sein können oder auch, gegebenenfalls partiell, hydriert sein können und Substituenten, vorzugsweise des Typs R¹, tragen können, mit der Maßgabe, daß die Zahl der Substituenten nicht größer ist als die Gesamtzahl der C-Atome;
- n: ist, gleich oder verschieden, 0,1,2,3,4,5;
- A, B: sind, gleich oder verschieden, Gruppen der Formel

(-M)ₐ(-E)_{b}(-M)_{c}-(-E)_{d}(-M)ₑ(-E)_{f}(-M)_{g}(-E)ₕ-R⁴
wobei die Symbole und Indizes folgende Bedeutungen haben:
- M: ist, gleich oder verschieden -CR⁵=CR⁶, -C≡C-, -CR⁷=N-, -N=CR⁷-;
- E: ist, gleich oder verschieden, Pyrazin-2,5-diyl, Pyridazin-3,6-diyl, Pyridin-2,5-diyl, Pyrimidin-2,5-diyl, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Naphthalin-2,6-diyl, Naphthalin-1,4-diyl oder Naphthalin-1,5-diyl, wobei eine oder zwei CH-Gruppen durch N ersetzt sein können, 1,3-Oxazol-2,4-diyl, 1,3-Oxazol-2,5-diyl, (1,3,4)-Oxadiazol-2,5-diyl, 4,4'-Biphenylen, Anthracen-diyl, Carbazol-diyl, Benzoxazol-diyl, Inden-2,5-diyl, Inden-2,6-diyl, wobei in den Ringsystemen ein oder mehrere H-Atome durch Reste R⁸ substituiert sein können;
- R⁴,R⁵,R⁶,R⁷: sind gleich oder verschieden
a) Wasserstoff, -F, -Cl, -CF₃, -CN, NR⁹R¹⁰
b) ein geradkettiger oder verzweigter Alkylrest (mit oder ohne asymmetrisches C-Atom) mit 1 bis 20 C-Atomen, wobei
   b1) eine oder mehrere nicht benachbarte und nicht terminale CH₂-Gruppen durch -O-, -S-, -CO-, -CO-O-, -O-CO-, -O-CO-O- oder -Si(CH₃)₂- ersetzt sein können und/oder
   b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
   b3) ein oder mehrere H-Atome durch F, CN und/oder Cl ersetzt sein können;
- R⁸: ist gleich oder verschieden
a) -F, -Cl, -CF₃, -CN, NO₂-
b) ein geradkettiger oder verzweigter Alkylrest (mit oder ohne asymmetrisches C-Atom) mit 1 bis 20 C-Atomen, wobei
   b1) eine oder mehrere nicht benachbarte und nicht terminale CH₂-Gruppen durch -O-, -S-, -CO-, -CO-O-, -O-CO-, -O-CO-O-, -NH-, N(C₁-C₁₀-Alkyl), -N-Phenyl-, -N-Tolyl-, -N(C₂H₅-OCH₃)- oder -Si(CH₃)₂- ersetzt sein können und/oder
   b2) eine oder mehrere CH₂-Gruppen durch -CH=CH-, -C≡C-, 1,4-Phenylen ersetzt sein können und/oder
   b3) ein oder mehrere H-Atome durch F, CN und/oder Cl ersetzt sein können;
- R⁹, R¹⁰: sind gleich oder verschieden
a) Wasserstoff
b) ein geradkettiger oder verzweigter Alkylrest (mit oder ohne asymmetrisches C-Atom) mit 1 bis 20 C-Atomen, wobei
   b1) eine oder mehrere nicht untereinander oder dem Stickstoff benachbarte CH₂-Gruppen durch -O-, -S-, -CO-, -CO-O-, -O-CO-, -O-CO-O-, oder -Si(CH₃)₂- ersetzt sein können und/oder
   b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
   b3) ein oder mehrere H-Atome durch F, CN und/oder Cl ersetzt sein können und
   b4) R⁸ und R⁹ zusammen auch einen Ring bilden können;
a, b, c, d, e, f, g, h sind unabhängig voneinander 0 oder 1. Vorzugsweise beträgt die Summe der Indizes mindestens 1, besonders bevorzugt mindestens 2. Besonders bevorzugt ist die Summe b+d+f+h ≥ 1, ganz besonders bevorzugt ≥ 2.

Besonders bevorzugte Verbindungen der Formel (1) sind Verbindungen der Formeln (IV), (V) und (VI), 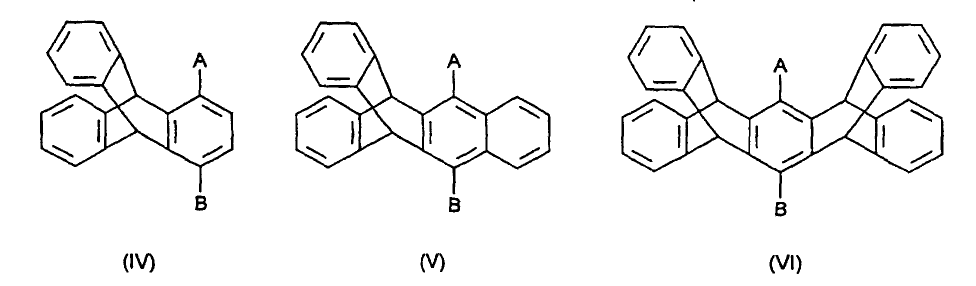 wobei die Gruppen A und B die oben angegebenen Bedeutungen haben.

Besonders bevorzugte Verbindungen der Formeln (IV), (V) und (VI) sind solche der Formeln (IV), (V), (VI) a-i 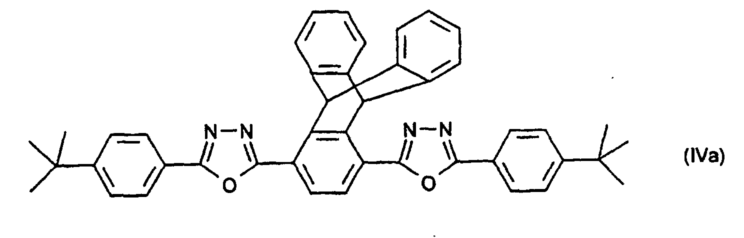 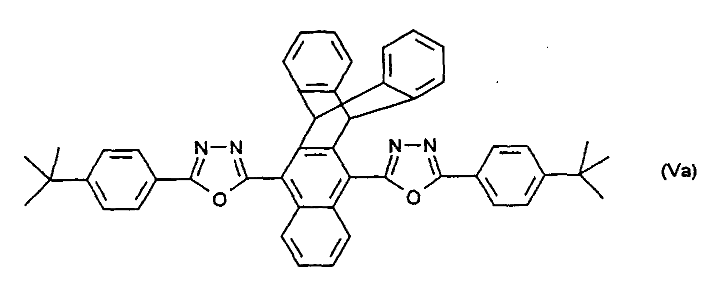 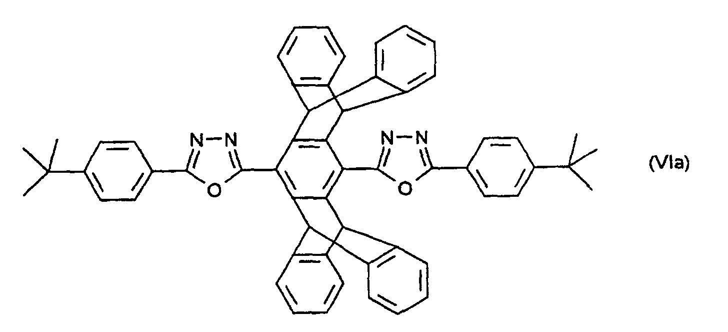 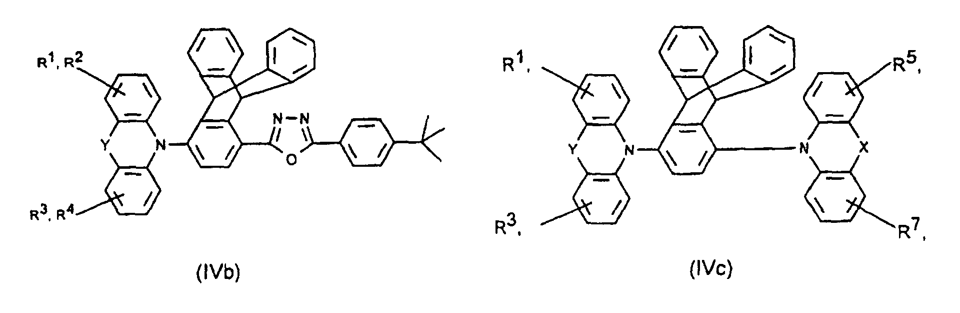 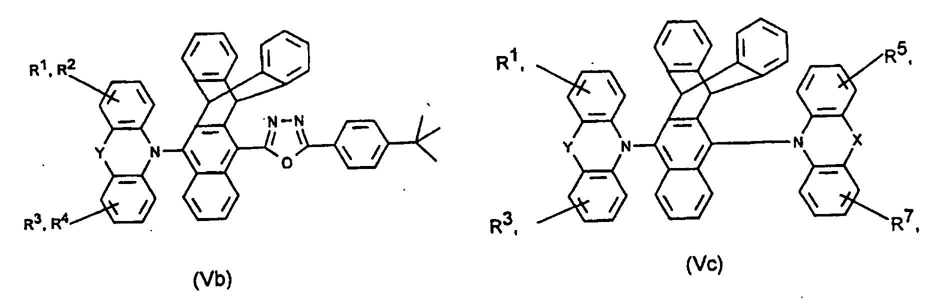 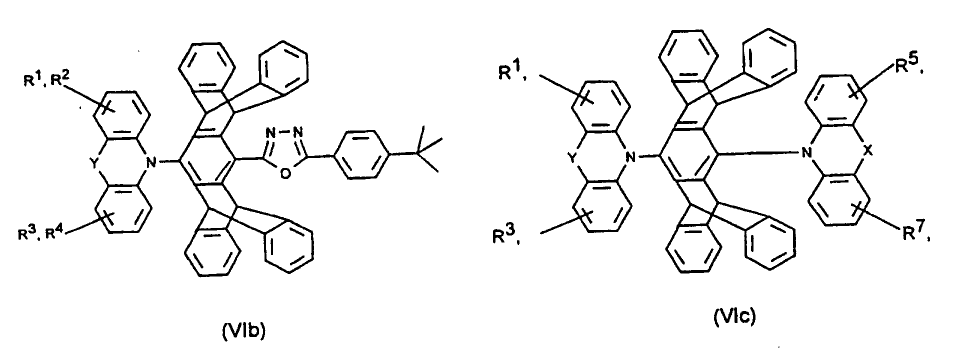 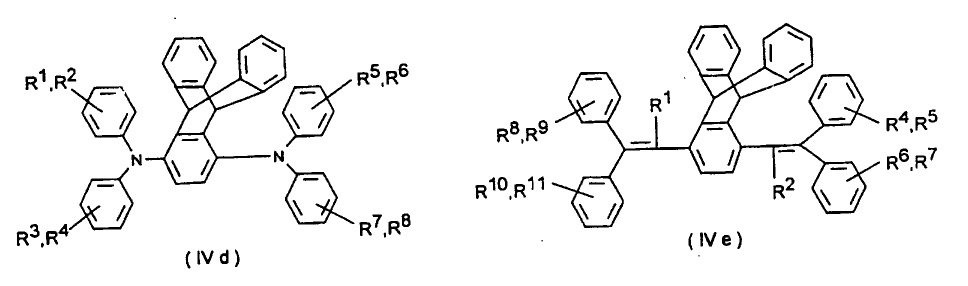 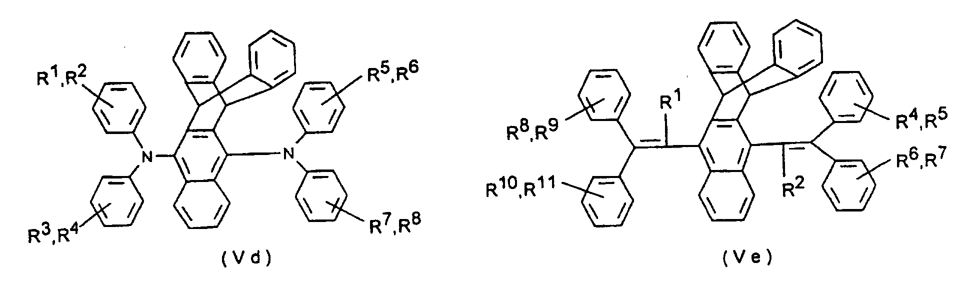 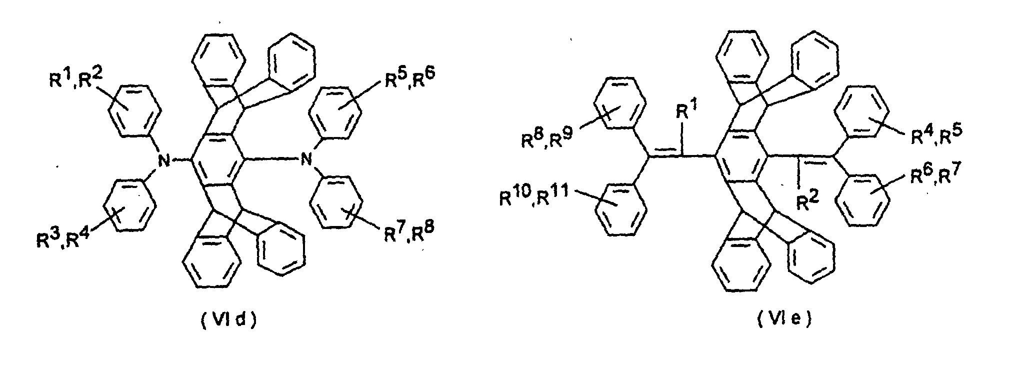 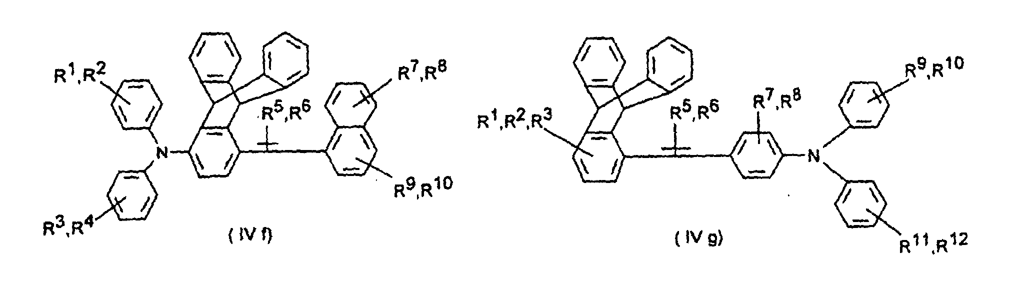 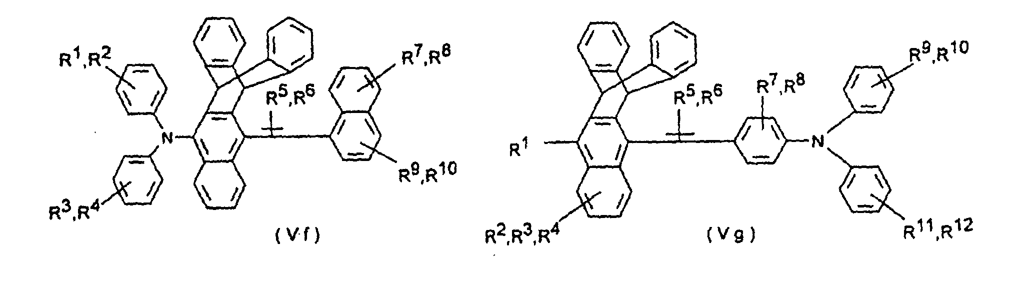 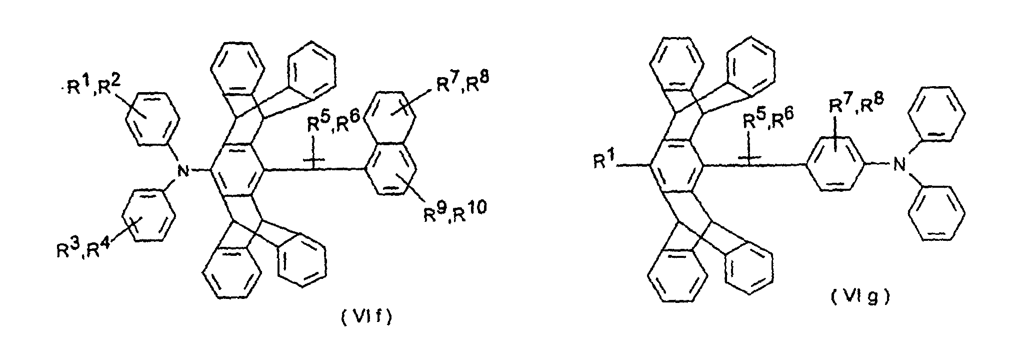 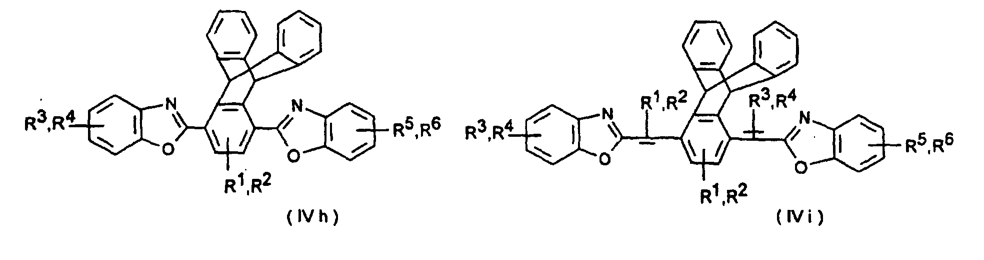 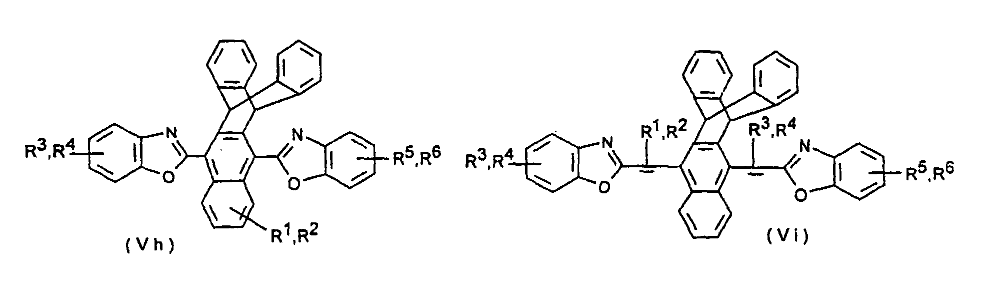 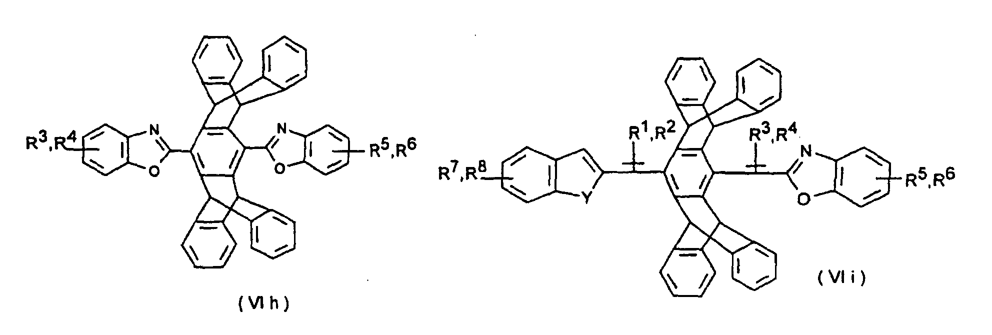 wobei die Reste R¹⁻¹¹ die gleichen Bedeutungen wie R³ in den Formeln (II) und (III) haben und Y: O,S,NR¹¹ oder CR¹⁰R¹¹ ist.

Gegenstand der Erfindung sind auch Triptycenderivate, bei denen mindestens eine der Gruppen K¹⁻³ ein Flurophor ist. Ebenso Gegenstand der Erfindung sind Triptycenderivate der Formel (II) und (III), bei denen die Summe der Indices a-h mindestens 1, vorzugsweise mindestens 2 beträgt.

Die Herstellung der erfindungsgemäßen Triptycenverbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart und in den entsprechenden Bänden der Serie "The Chemistry of Heterocyclic Compounds" von A. Weissberger und E.C. Taylor (Herausgeber) beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Verbindungen der Formel (II) können beispielsweise ausgehend vom substituierten Triptycen bzw. Heterotriptycengrundkörper synthetisiert werden, welcher wiederum über verschiedene Synthesewege zugänglich ist. Exemplarisch, aber nicht einschränkend seien an dieser Stelle genannt:
1. Synthesen aus substituierten Anthracen (bzw. substituiertem Acridin oder substituiertem Phenazin) und Dehydroaromaten z.B. ausgehend von
   a) substituierten o-Fluorbrombenzolen mit reaktiven Metallen wie z.B. Magnesium, z.B. analog zu G.Wittig, Org. Synth. IV 1963, 964;
   b) substituierten o-Dihalogenbenzolen und Butyllithum unter Metallhalogenideliminierung, z.B. analog zu H. Hart, S. Shamouilian, Y. Takehira J.Org.Chem. 46 (1981) 4427;
   c) substituierten Monohalogenbenzolen und starken Basen unter Halogenwasserstoffeliminierung, z.B. anlog zu P. G. Sammes, D. J. Dodsworth, J.C.S.Chem. Commun. 1979, 33.
   d) substituierten Anthranilsäurederivaten und Isoamylnitril, z.B. analog zu C.W. Jefford, R.McCreadie, P. Müller, B. Siegfried, J.Chem.Educ. 48 (1971) 708.
   e) eine Übersicht zur Herstellung einer Reihe von substituierten Dehydroaromaten findet sich in Houben-Weyl, Methoden der Organischen Chemie, 4. Auflage 1981, Band V/2b, pp. 615, Georg-Thieme-Verlag, Stuttgart.
2. Synthesen durch Desaminierung substituierter Anthracen-9,10-imine z.B. analog zu L. J. Kricka, J. M. Vernon, J.C.S. Perkin I, 1973, 766.
3. Synthese durch Cycloaddition von substituierten 1,4-Chinonen mit substituierten Anthracenenderivaten z.B. analog zu E.Clar, Chem.Ber. 64 (1931) 1676; W.Theilacker, U. Berger-Brose, K.H. Beyer, Chem.Ber. 93 (1960) 1658; P.D. Bartlett, M.J. Ryan, J. Am.Chem. Soc. 64 (1942) 2649; P. Yates, P. Eaton, J.Am.Chem.Soc. 82 (1960) 4436. V.R. Skvarchenko, V.K. Shalaev, E.I. Klabunovskii, Russ.Chem.Rev. 43 (1974) 951;

Weiter Synthesen substituierter Triptycene finden sich exemplarisch in C.F. Wilcox, F.D. Roberts, J.Org.Chem. 30 (1965) 1959; T.H. Regan, J.B. Miller, J.Org.Chem. 32 (1967) 2798.

Weitere Synthesen für Heterotriptycene finden sich beispielsweise in D.Hellwinkel. W.Schenk, W.Blaicher, Chem.Ber. 111 (1978) 1798; oder D.Hellwinkel, W.Schenk, Angew.Chem. 24 (1969) 1049; N.P. McCleland, J.B. Withworth, J.Am.Chem.Soc. (1927) 2753; N.A.A. Al-Jabar, A.G. Massey, J.Organomet.Chem. 287 (1985) 57.

Verbindungen der Formel (III) können beispielsweise ausgehend vom substituierten Bis-triptycengrundkörper bzw. Hetero-bistrypticengrundkörper synthetisiert werden, welcher wiederum über verschiedene Synthesewege zugänglich ist. Exemplarisch, aber nicht einschränkend seien an dieser Stelle genannt:
1) Synthesen aus substituierten Anthracen (bzw. substituiertem Acridin oder substituiertem Phenazin) und substituierten Didehydrobenzolen z.B. analog zu H. Hart, S. Shamouilian, Y. Takehira J.Org.Chem. 46 (1981) 4427;
2) Synthese durch Cycloaddition von substituierten Anthracenenderivaten mit 1,4-Benzochninon z.B. analog zu E.Clar, Chem.Ber. 64 (1931) 1676; P. Yates, P. Eaton, J.Am. Chem. Soc. 82 (1960) 4436; W.Theilacker, U. Berger-Brose, K.H. Beyer, Chem.Ber. 93 (1960) 1658.

Weitere Synthesen finden sich exemplarisch in H. Hart, A. Bashir-Hashemi, J. Luo, M.A. Meador, Tetrahedron 42 (1986) 1641; V.R. Skvarchenko, V.K. Shalevb, E.I. Klabunovskii, Russ.Chem.Rev. 43 (1974) 951; V.R. Skvarchenko, A.G. Shil'nikova, N.N. Konrat'eva, R.Ya. Levina, J.Org.Chem. USSR (engl.trans.) 3 (1967) 1477.

Die weitere Funktionalisierung der oben beschriebenen Grundkörper kann beispielsweise ausgehend von den entsprechenden 1,4-Dialkyl-bzw. 1,4-Dimethylderivaten erfolgen, wobei durch Halogenierung bzw. Oxidation zum Aldehyd bzw. zur Carbonsäure die entsprechenden Gruppierungen für den Aufbau der Gruppen A und B in den Verbindungen der allgemeinen Formeln (II) und (III) entstehen. Als beispielhafte Synthesen seien genannt: Zaug, Rapalla, Org.Synth. 27 (1947) 84; Trahanovsky, Young, J.Org.Chem. 31 (1996) 2033; San Filippo, J.Org.Chem. 42 (1977) 2182; Étard, Ann.,Chim.Phys. 22 (1881) 218; Sharpless J.Am. Chem. Soc. 97 (1975) 5927.

Ausgehend von den Triptycen- bzw. Bistriptycenchinonen ist ebenfalls eine Einführung von Gruppierungen für den Aufbau der Gruppen A und B in den Verbindungen der allgemeinen Formeln (II) und (III) zu erreichen. So lassen sich durch Reaktion der Chinone mit metallorganischen Reagenzien mit anschließender zweifacher Dehydroxylierung verschienden Gruppierungen wie z.B. Alkyl, Aryl, Alkin, einführen, als Beispiele für entsprechend analoge Reaktionen seien angegeben: T. Imamoto, N. Takiyama, K. Nakamura, T. Hatajima, Y. Kamiya, J.Am.Chem.Soc., 111 (1989) 4392; A.Fischer, G.N. Henderson, Tetrahedron Lett. 24 (1983) 131; H.M. Crawford, J.Am.Chem.Soc. 70 (1948) 1081; C.T. Wigal, J.D. McKinley, J.Coyle, D.J.Porter, D.E. Lehman, J.Org.Chem. 60 (1995) 8421; bzw. entsprechende Kapitel in Houben-Weyl, Methoden der Organischen Chemie, Georg-Thieme-Verlag, Stuttgart. Für die zweifache Dehydroxylierung die sehr effektiv mit niedervalentem Titan oder Zinn bzw. Phenylhydrazin erfolgen kann, seien beispielhaft genannt; G. Solladie, A. Girardin, J.Org.Chem. 54 (1989) 2620 bzw. M. lyoda, T.Yamauchi, M.Oda J.C.S. Chem.Commun., 1986, 303; K.J.Clark, J.Chem.Soc. (1956) 1511.

Ausgehend von den Triptycen- bzw. Bistriptycenhydrochinonen ist nach Überführung in die entsprechenen Triflate, Mesylate oder Nonaflate sowohl eine palladiumkatalysierte Kupplung bzw. Polymerisation durchführbar z.B. mit zinnorganischen Verbindungen analog zu Z. Bao, W.K.Chan, L.Yu, J.Am.Chem.Soc. 117 (1995) 12426; A.M. Echavarren, J.K.Stille, J.Am.Chem.Soc 109 (1987) 5478. K. Ritter, Synthesis (1993) 735. Weiterhin ist die Kupplung der aus den Hydrochinonen leicht zugänglichen Triflate bzw. Mesylate mit Organoboronsäuren möglich, z.B. analog zu T.Oh-e, N.Miyaura, A.Suzuki, Synlett (1990) 221; A.R. Martin, Y. Yang, Acta Chem.Scand. 47 (1993) 221; J.M.Fu, V.Sniekus, Tetrahedron Lett. 29 (1988) 1665; V. Percec, S.Okita, J.Polym.Sci.A 31 (1993) 877. Zusätzlich sind die Triflate bzw. Mesylate Edukte für eine Nickel-katalysierte C-C-Kupplung z.B. analog zu V. Percec, J.Y. Bae, M. Zhao, D.H. Hill, J.Org.Chem. 60 (995) 176; V.Percec, C.Pugh, E.Cramer, S.Okita, R.Weiss; Macromol.Symp. 54/55 (1992) 113. V.Percec, S.Okita, R.Weiss, Macromolecules 25 (1992) 181.6; Y. Yamashita, Y. Inoue, T.Kondo, H.Hashimoto, Chem.Lett. (1986) 407;

Für die Synthese der Gruppen A und B, sei weiterhin beispielsweise verwiesen auf J.S. Schumm, D.L. Pearson, J.M. Tour, Angew.Chem. 106 (1994) 1445; bzw. auf DE-A 23 44 732, 24 50 088, 24 29 093, 25 02 904, 26 36 684, 27 01 591 und 27 52 975 für Verbindungen mit 1,4-Phenylen-Gruppen; DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen;DE-A 32 31 462 für Verbindungen mit Pyridazin-3,6-diyl-Gruppen; N. Miyaura, T. Yanagi und A, Suzuki in Synthetic Communications 11 (1981) 513 bis 519, DE-C-3 930 663, M.J. Sharp, W. Cheng, V. Snieckus in Tetrahedron Letters 28 (1987), 5093; G.W. Gray in J.Chem.Soc. Perkin Trans II, (1989) 2041 und Mol.Cryst.Liq.Cryst. 172 (1989) 165, Mol.Cryst.Liq.Cryst. 204 (1991) 43 und 91; EP-A 0 449 015; WO 89/12039; WO 89/03821; EP-A 0 354 434 für die direkte Verknüpfung von Aromaten und Heteroaromaten;

Die Herstellung disubstituierter Pyridine, disubstituierter Pyrazine, disubstituierter Pyrimidine und disubstituierter Pyridazine findet sich beispielsweise in den entsprechenden Bänden der Serie "The Chemistry of Heterocyclic Compounds" von A. Weissberger und E.C. Taylor (Herausgeber).

Die erfindungsgemäßen Verbindungen der Formel (I) eignen sich zur Verwendung als Elektrolumineszenzmaterialien.

Als Elektrolumineszenzmaterialien im Sinne der Erfindung gelten Materialien, die als oder in einer aktiven Schicht in einer Elektrolumineszenzvorrichtung Verwendung finden. Aktive Schicht bedeutet, daß die Schicht befähigt ist, bei Anlegen eines elektrischen Feldes Licht abzustrahlen (lichtemittierende Schicht) und/oder daß sie die injektion und/oder den Transport der positiven und/oder negativen Ladungen verbessert (Ladungsinjektions- oder Ladungstransportschicht). Darüber hinaus ist auch die Verwendung als Elektronenblockierschicht oder Lochblockierschicht erfindungsgemäße Anwendung

Gegenstand der Erfindung ist daher auch die Verwendung einer Triptycenderivats der Formel (I) als Elektrolumineszenzmaterial.

Um als Elektrolumineszenzmaterialien Verwendung zu finden, werden die Triptycenderivate der Formel (I) im allgemeinen nach bekannten, dem Fachmann geläufigen Methoden, wie Eintauchen (Dipping), Lackschleudem (Spincoating), Aufdampfen oder Auspuffern im Vakuum, in Form eines Films auf ein Substrat aufgebracht.

Gegenstand der Erfindung ist somit ebenfalls eine Elektrolumineszenzvorrichtung mit einer oder mehreren aktiven Schichten, wobei mindestens eine dieser aktiven Schichten ein oder mehrere Triptycenderivate der Formel (I) enthält. Die aktive Schicht kann beispielsweise eine lichtemittierende Schicht und/oder eine Ladungstransportschicht und/oder eine Ladungsinjektionsschicht sein.
Der allgemeine Aufbau solcher Elektrolumineszenzvorrichtungen ist beispielsweise in US 4,539,507 und US 5,151,629 beschrieben.

Sie enthalten üblicherweise eine elektrolumineszierende Schicht zwischen einer Kathode und einer Anode, wobei mindestens eine der Elektroden zumindest für einen Teil des sichtbaren Spektrums transparent ist. Zusätzlich können zwischen der elektrolumineszierenden Schicht und der Kathode eine oder mehrere Elektroneninjektions- und/oder Elektronentransportschichten eingebracht sein und/oder zwischen der elektrolumineszierenden Schicht und der Anode eine oder mehrere Lochinjektions- und/oder Lochtransportschichten eingebracht sein. Als Kathode können vorzugsweise Metalle oder metallische Legierungen, z.B. Ca, Mg, Al, In, Mg/Ag dienen. Als Anode können Metalle, z.B. Au, oder andere metallisch leitende Stoffe, wie Oxide, z.B. ITO (Indiumoxid/Zinnoxid) auf einem transparentem Substrat, z.B. aus Glas oder einem transparenten Polymer, dienen.

Im Betrieb wird die Kathode auf negatives Potential gegenüber der Anode gesetzt. Dabei werden Elektronen von der Kathode in die Elektroneninjektionsschicht-/Elektronentransportschicht bzw. direkt in die lichtemittierende Schicht injiziert. Gleichzeitig werden Löcher von der Anode in die Lochinjektionsschicht/Lochtransportschicht bzw. direkt in die lichtemittierende Schicht injiziert.

Die injizierten Ladungsträger bewegen sich unter dem Einfluß der angelegten Spannung durch die aktiven Schichten aufeinander zu. Dies führt an der Grenzfläche zwischen Ladungstransportschicht und lichtemittierender Schicht bzw. innerhalb der lichtemittierenden Schicht zu Elektronen/Loch-Paaren, die unter Aussendung von Licht rekombinieren.
Die Farbe des emittierten Lichtes kann durch die als lichtemittierende Schicht verwendeten Materialien variiert werden.

Elektrolumineszenzvorrichtungen finden Anwendung z.B. als selbstleuchtende Anzeigeelemente, wie Kontrollampen, alphanumerische Displays, Hinweisschilder, und in optoetektronischen Kopplem.

Verbindungen der Formel (I) eignen sich weiterhin beispielsweise zur Verwendung in optischen Speichern, als photorefraktive Materialien, für nichtlinear-optische (NLO) Anwendungen, als optische Aufheller und Strahlungskonverter und, bevorzugt, als Lochtransportmaterialien in photovoltaischen Zellen, wie sie z.B. in WO-A 97/10 617 und DE-A 197 11 713 beschrieben, auf die hier ausdrücklich Bezug genommen wird.

Die Erfindung wird durch die Beispiele näher erläutert, ohne sie dadurch beschränken zu wollen.

### Beispiel 1: Synthese von Dihydrotriptycen-1,4-chinon

17.8 g (100 mmol) Anthracen und 10.8 g (100 mmol) *p*-Benzochinon (frisch sublimiert) wurden unter Stickstoffüberlagerung bei 135°C in 200 ml *p*-Xylol gelöst. Nach einigen Minuten begann sich aus der nun rot gefärbten Lösung ,ein gelber, kristalliner Niederschlag abzuscheiden. Nach 4 Stunden ließ man auf Raumtemperatur abkühlen und saugte den Niederschlag ab. Der gelbe Feststoff wurde mit *p*-Xylol nachgewaschen und im Vakuum getrocknet. Die erhaltenen 26.0 g (91 mmol, 91 %) wurden in 100 ml *p*-Xylol unter N₂ 0.5 Stunden lang auf 130°C erhitzt gehalten, auf Raumtemperatur abgekühlt, abgesaugt, mit Methanol nachgewaschen und getrocknet. Man erhielt 23.5 g (82 mmol, 82 %) Dihydrotriptycen-1,4-chinon als fahl-gelbe Kristalle
Schmelzpunkt: 232°C
¹H NMR: (400 MHz; CDCl₃): δ [ppm] =3.15 (t, 2H, tert. H), 4.86(s, 2H, Enyl-H), 6.30(s, 2H, Brückenkopf-H) 7.07 und 7.39(4H, m, *J* = 5.3 Hz, 2.3 Hz -Phenyl-H), 7.17-7.20ppm, m, 4H, Phenyl-H ).

### Beispiel 2: Synthese von 1,4-Triptycen-1,4-chinon

37.0 g (129 mmol) Dihydrotriptycen-1,4-chinon wurden in 350 ml Eisessig suspendiert und in der Siedehitze mit 1.5 ml HBr (48%ig in Wasser) versetzt. Es wurde zwei Stunden zum Rückfluß erhitzt. In der Siedehitze wurde dann eine Lösung von 13.0g KIO₃ (60 mmol) innerhalb von 5 Minuten zugetropft. Sofort zeigt sich eine Gelbfärbung der Suspension. Man ließ erkalten, setzte bei 50°C noch 200 ml Wasser zu und saugte ab. Dann wurde mehrmals mit Na₂SO₃-Lösung, anschließend mehrmals mit Wasser gewaschen und im Vakuum getrocknet. Das Rohprodukt (35.2g, 96%) wurde 2x je eine Stunde in 150 ml Isopropanol trituriert. Man erhielt 30.9 g (108.7 mmol, 84 %) 1,4-Triptycen-1,4-chinon als leuchtend gelbe pulvrige Substanz.
Schmelzpunkt: 273-275°C
¹H NMR: (400 MHz;CDCl₃): δ[ppm] =5.79 (s, 2H, Brückenkopf-H), 6.59(s, 2H, Enyl-H), 7.03 und 7.42 (m, 8H, *J* = 2.3 Hz, 5.3Hz, AB-System Phenyl-H)

### Beispiel 3: Synthese von 1,4-Dihydroxy-1,4-dimethyltriptycen

In einem 1l Vierhalskolben wurden 148 ml (237 mmol, 2.7 eq) einer 1.6 M Lösung von Methyllithium in Diethylether mit 300 ml THF (destilliert von Na/Benzophenon) vorgelegt und auf -78°C gekühlt (Aceton/Trockeneis). Gleichzeitig wurde eine Lösung von 25.0 g (87.9 mmol) 1,4-Triptycen-1,4-chinon in 600 ml THF auf dieselbe Temperatur abgekühlt. Die Lösung des 1,4-Triptycen-1,4-chinons wurde in einen Tropftrichter überführt, der mittels Trockeneis zusätzlich gekühlt wurde. Unter kräftigem Rühren wurde die Eduktlösung langsam zugetropft (1 Stunde), wobei sich die Lösung sofort blau bis blaugrün färbte. Nach beendeter Zugabe wurde die Temperatur noch eine weitere Stunde gehalten und anschließend die Kühlung entfernt. Man ließ auf Raumtemperatur erwärmen und rührte über Nacht.
Die Suspension wurde im Vakuum auf etwa 200 ml eingeengt und anschließend auf eine Mischung aus 1.4 L Eiswasser und 10 g NH₄Cl gegossen. Beim Eingießen trat Wärmeentwicklung auf und es fiel ein hellbeiger Niederschlag aus, der sich beim Erwärmen auf Raumtemperatur verflüssigte. Das resultierende Öl wurde abgetrennt und die Wasserphase wurde 3 x mit 500 ml CH₂Cl₂ extrahiert. Die vereinigten organische Phase wurden zweimal mit je 200 ml Wasser gewaschen, mit Na₂SO₄ getrocknet und so weit wie möglich einrotiert.
Es verblieb eine braune, zähe Masse, die mit 30 ml Diethylether/Hexan 2:1 solange im Ultraschallbad behandelt wurde, bis alles Öl in Lösung gegangen war und sich ein weißer Niederschlag gebildet hatte. Der Niederschlag wurde abgesaugt und die Mutterlauge wurde erneut einrotiert und auf die gleiche Art und Weise behandelt, wobei die Volumina der Et₂O/Hexan- Mischung immer etwas kleiner gewählt wurden. Es wurde analog verfahren, bis kein Niederschlag mehr ausfiel. Zur weiteren Reinigung wurde das Reaktionsgemisch in Diethylether refluxiert, auf 20°C gekühlt und abgesaugt. Man erhielt 14.9 g (47.1 mmol, 54%) 1,4-Dihydroxy-1,4-dimethyltriptycen als weißes Pulver.
¹H NMR: (400 MHz; DMSO-d₆):δ =1.09 (s, 6H, Methyl-H); 4.84 (s, 2H, Hydroxy-H); 5.34 (s, 2H, Chinon-H); 5.63 (s, 2H, Brückenkopf-H); 6.90, 6.92, 7.28, 7.33 (m, je 2H, *J*=5.3 Hz und 2.3 Hz, Phenyl-H).
¹H NMR: (400 MHz; CDCl₃):δ =1.27 (s, 6H, Methyl-H); 1.63 (s, 2H, Hydroxy-H); 5.39 (s, 2H, Chinon-H); 5.54 (s, 2H, Brückenkopf-H); 6.91 (m, 2H, *J*=5.5 Hz und 2.3 Hz, Phenyl-H); 6.95 m, 2H, *J*=5.3 Hz und 2.0 Hz, Phenyl-H); 7.32(m, 4H, *J*=5.3Hz, 2.3 Hz und 2.0 Hz, Phenyl-H).

### Beispiel 4: Synthese von 1,4-Dimethyltriptycen

6.70 g (53.3 mmol, 2.1 eq) SnCl₂· 2 H₂O wurden in 200 ml 50%iger Essigsäure gelöst. Dazu wurde eine methanolische Lösung von 8.44 g (25.7 mmol) 1,4-Dihydroxy-1,4-dimethyltriptycen so langsam zugetropft, daß die Temperatur nicht höher als max. 45°C anstieg. Die Reaktionslösung wurde gelblich und ein weißer Niederschlag schied sich ab. Nach erfolgter Zugabe wurde weitere 2h bei Raumtemperatur gerührt. Danach kühlte man auf -18°C und saugte den erhaltenen Niederschlag ab, wusch ihn mit ca. 1L Wasser säurefrei und trocknete im Vakuum.

Die erhaltene Mutterlauge wurde danach etwas einrotiert und der nach erneutem Abkühlen ausgefallenen Niederschlag wiederum abgesaugt. Es wurde 7.0 g Rohprodukt erhalten. Die Verbindung wurde in etwa 300 ml Aceton in der Siedehitze gelöst und anschließend mit 50 ml Wasser gefällt. Die Lösung wurde im Eisfach gekühlt und der Niederschlag wurde abgesaugt. Nach erneuter Durchführung der Prozedur erhielt man 5.20 g (18.4 mmol, 72 %) 1,4-Dimethyltriptycen als weiße Kristallflitter.
Schmelzpunkt: 246-249°C
¹H NMR: (400 MHz; DMSO-d₆):δ =2.43 (s, 6H, Methyl-H); 5.80 (s, 2H, Brückenkopf-H); 6.71 (s, 2H, Phenyl-H); 6.98 und 7.45 (m, 8H, *J* = 2.3Hz, 5.3Hz, AB System, Phenyl-H).
¹H NMR: (400 MHz; CDCl₃): δ =2,46 (s, 6H, Methyl-H); 5.64 (s, 2H, Brückenkopf-H); 6.70 (s, 2H, Phenyl-H); 6.97 und 7.36 (m, 8H, *J* = 2.3Hz, 5.3Hz, AB-System, Phenyl-H).

### Beispiel 5: Synthese von 1,4-Bis-(brommethyl)-triptycen

4.9g (17.5mmol) Dimethyltriptycen wurden in 150ml trockenem Tetrachlorkohlenstoff gelöst und mit 7.2g (40mmol) NBS, sowie 1 mol% AZIBN versetzt. Die Suspension wurde unter Bestrahlung mit einer Hg-Lampe zum gelinden Rückfluß erhitzt. Danach ließ man erkalten und trennte vom Succinimid ab. Die Tetrachlormethanlösung wurde einrotiert und der Rückstand zweimal aus Acetonitril umkristallisiert.. Man erhielt 7.7g (96%) farbloses 1,4-Bis-(brommethyl)-triptycen vom Schmelzpunkt 198-204°C.
¹H NMR; 400 MHz/CDCl₃:
δ/ppm = 4.67 (s, 4H), 5.4 (s, 2H), 6.9 (s, 2H), 7.02 und 7.47 (je m, je 4H);
IR (KBr):ν/cm⁻¹: 3034, 2987, 1460, 1444, 1403, 1202, 822, 716, 616;
MS: (FD, 8kV); m/z : 439.9 (100%),[M⁺];

### Beispiel 6:

### Synthese von 6,11-Dioxo-5,5a,11a,12-tetrahydro-5, 12-o-benzeno-naphthacen

In einem 2000ml Zweihalskolben wurden 45.0g (250mmol) Anthracen und 40.0g (250mmol) vorgereinigtes (Et₂O/Aktivkohle) 1,4-Naphtochinon in 700ml trockenem CHCl₃ suspendiert und mit Eis auf 0°C abgekühlt. Anschließend wurden 34.7g (260mmol) wasserfreies Aluminiumchlorid auf einmal zur Suspension gegeben. Dabei verfärbte sich die Reaktionslösung von anfänglich rötlich nach tiefblau. Nach erfolgter Zugabe wurde das Eisbad entfernt. Es war sofort ein Farbumschlag nach hellbeige bis grün erkennbar. Man rührte bei Raumtemperatur noch 2 Stunden weiter.
Danach wurden 500ml CHCl₃ hinzugefügt und die Reaktionslösung auf 700ml Eis/Wasser gegossen.
Der dabei ockerfarben ausgefallene Niederschlag wurde durch Zugabe von so wenig wie möglich Chloroform bei Raumtemperatur wieder in Lösung gebracht. Die Phasen wurden in einem Scheidetrichter getrennt, die Wasserphase zweimal mit je 200ml CHCl₃ extrahiert, 2x mit 100ml gesättigter Natriumhydrogencarbonatlösung und 1x mit 100ml Natriumchloridlösung gewaschen und die vereinigten Extrakte über wasserfreiem Na₂SO₄ getrocknet. Beim Einengen des Lösemittels wurden an dem Punkt, an dem Kristallisation beginnt 400 ml MeOH hinzugefügt, woraufhin das Produkt auskristallisierte.
Nach erneutem Lösen in Chloroform und Fällen mit Methanol erhielt man 84.0g (96 %) farblos, kristallines 6,11-Dioxo-5,5a,11a,12-tetrahydro-5, 12-o-benzeno-naphthacen vom Schmelzpunkt 207-208°C.
¹H-NMR; 400MHz/CDCl₃:
δ=3.37 ( t, 2 H), 5.00 (s, 2 H), 7.10-7.12, 7.18-7.20, 7.42-7.45, 7.54-7.56, 7.83-7.86 (je m, 2H);
IR(KBr): v/cm⁻¹: 3063, 3020, 2963, 1676, 1589, 1467, 1270, 1005, 758,727, 574;

### Beispiel 7: Synthese von 6,11-Dioxo- 5,12-dihydro-5,12-o-benzeno-naphthacen (Naphtotripycen-6,11-chinon)

16.0 g (47.5 mmol) Dihydronaphtotriptycen-6,11-chinon wurden in einem 1 L Kolben in 500 ml Eisessig in der Siedehitze gelöst und mit 1.5 ml 48% HBr in Wasser versetzt. Nach etwa 25 Minuten bei 100°C wurde eine Lösung von 2.65 g (15.8 mmol) Kaliumbromat in 120 ml Wasser langsam zugetropft. Beim Zutropfen setzte zuerst ein Farbumschlag nach gelb ein, der aber sofort mit einer Schwärzung der entstehenden Suspension in Verbindung mit einer Eindickung des Reaktionsgemisches einherging. Nachdem alles KBrO₃ zugegeben war, wurde weitere 5 Stunden bei 100 °C gehalten. Die Farbe veränderte sich nach gelb. Es wurde dann in der Hitze mit Wasser auf 1000 ml Volumen aufgefüllt und das Heizbad entfernt. Nach Erkalten auf Raumtemperatur wurde abgesaugt, mit Wasser neutral gewaschen und getrocknet. Es wurden 15.0 g (94 %) Naphtotriptycen-6,11-chinon als leuchtend gelbe kleine Kristalle erhalten.
Schmelzpunkt: 295-300°C
¹H NMR (CDCl₃, 400 MHz): δ= 6.00 (s, 2H, Brückenköpfe); 7.03-7.05 (m, 4H, Phenyl-H); 7.45-7.47 (m, 4H, Phenyl-H); 7.64-7.67 (m, 2H, Phenyl-H); 8.03-8.06 (m, 2H, Phenyl-H).

### Beispiel 8: Synthese von 6,11-Dihydroxy-6,11-dimethyl-5,12-dihydro-5,12-o-benzeno-naphthacen

210ml (470mmol) Methyllithium (2.2 M in Ether/Hexan) wurden zusammen mit 50 ml THF in einem 2l Vierhalskolben vorgelegt und mit Aceton/Trockeneis auf -78°C gekühlt. Zu diesem Gemisch wurden langsam und unter starkem Rühren 60.0g (180mmol) *6,11-Dioxo-5,5a,11a,12-tetrahydro-5, 12-o-benzeno-naphthacen* in 700ml THF getropft. Nach der Zugabe wurde drei Stunden auf niedriger Temperatur gehalten. Dann ließ man während drei Stunden auf Raumtemperatur kommen und rührte weitere zwei Stunden bei Raumtemperatur. Es wurden etwa 400ml THF im Vakuum abdestilliert und die Suspension auf 2.5l Wasser/Eis (2:1) gegossen in dem 30g NH₄Cl gelöst war, so daß etwa pH 8 bestehenbleibt. Das ausgefallene Rohprodukt wurde abgesaugt und getrocknet.
Nach zweimaliger Umkristallisation aus Aceton/Wasser erhielt man 60.4g (79%) farbloses 6,11-Dihydroxy-6,11-dimethyl-5,12-dihydro-5,12-o-benzeno-naphthacen vom Schmelzpunkt 255-260°C (Zers.).
¹H-NMR; 400MHz/CDCl₃:
δ=1.40 (s, 6H), 1.82 (s, 2H), 5.55 (s, 2H), 6.92-6.94(m, 2H), 6.97-6.99 (m, 2H), 7.25-7.28 (m, 2H), 7.34-7.38 (m, 4H), 7.58-7.60 (m, 2H);
IR: v/cm⁻¹: 3582, 3400-3200, 3066, 3016, 2973, 2920, 1456, 1228, 918, 761, 652;

### Beispiel 9: Synthese von 6-Hydroxymethyl-11-Methyl-5,12-dihydro-5,12-o-benzeno-naphthacen (6-Methyl-11-hydroxymethylnaphtotriptycen) und 6-Chlormethyl-11-methyl-5,12-dihydro-5,12-o-benzeno-naphthacen (6-Methyl-11-chlorymethylnaphtotriptycen)

In einem 500 ml Dreihalskolben wurden unter N₂ 76 ml (145 mmol) TiCl₃ Lösung (1.9 M in 2N HCl) vorgelegt und in einer Aceton/Eis Mischung (-10°C) gerührt. Über einen Tropftrichter wurde eine Lösung von 13.3 g (36.3 mmol) Naphtotriptycen-6,11-dimethyl-6,11-diol in 200 ml DME getropft. Nach etwa 5 Minuten begann aus der Lösung ein weißer Niederschlag auszufallen, der auf der Reaktionsmischung schwamm. Nach fünf Stunden bei Raumtemperatur zeigt ein DC zwei Spots, die der Hydroxymethyl- und Chlormethylverbindung entspricht. Das ausgefallene 6-Methyl-11-chlormethylnaphtotriptycen wurde abgesaugt. Man erhielt 9.3g (70%) Rohprodukt.
Die Phasen des Filtrats wurden getrennt und die organische Phase nach Waschen mit 2 N HCl getrocknet (Na₂SO₄) und einrotiert. Der verbleibende Rückstand wurde in Methylenchlorid gelöst und nochmals mit 2 N HCl ausgeschüttelt. Diese Prozedur wurde sooft durchgeführt bis evtl. vorhandene Trübungen verschwunden sind (6 mal). Nach Trocknen wurde (Na₂SO₄) soviel Methylenchlorid abgezogen, bis in der Siedehitze gerade noch alles in Lösung blieb. Es wurden weitere 1.7g (13%) eines Gemisches aus 6-Methyl-11-hydroxymethylnaphtotriptycen und 6-Methyl-11-chlormethylnaphtotriptycen erhalten.
Dieses Gemisch wurde durch vierstündiges Refluxieren in einer Lösung von 4N HCl in Dioxan und anschließender Fällung der erkalteten Suspension mit kalter 2 N HCl in 6-Methyl-11-chlormethylnaphtotriptycen umgewandelt werden. Die Verbindung wurde aus Methylenchlorid / Hexan umkristallisiert.
Reines 6-Methyl-11-hydroxymethylnaphtotriptycen wurde durch mehrstündiges Rühren in N-Methylpyrrolidon / Wasser (2:1) bei 90°C erhalten.

6-Methyl-11-chloromethylnaphtotriptycen:
Schmelzpunkt: 308-312°C
¹H NMR (DMSO-d₆, 400 MHz): d = 2.83 (s, 3H, Methyl-H), 5.60 (s, 2H, Methylen-H), 6.18, 6.26 (s, je 1H, Brückenköpfe), 7.03-7.07 (m, 4H, Phenyl-H), 7.50-7.58 (m, 6H, Phenyl-H), 8.00-8.03 (m, 1H, Naphtyl-H), 8.08-8.11 (m, 1H, Naphtyl-H)
¹H NMR (CDCl₃, 400 MHz): d = 2.83 ( s, 3H, Methyl-H), 5.31 (s, 2H, Methylen-H), 5.91, 5.93 ( s, je 1H, Brückenköpfe), 7.03-7.06 (m, 4H, Phenyl-H), 7.41-7.48 (m, 6H, Phenyl-H), 7.94-7.96 ( m, 1H, Naphtyl-H), 7.99-8.01 (m, 1H, Naphthyl-H)
Masse: m/z:365.9 (M⁺)

6-Methyl-11-hydroxymethylnaphtotriptycen:
Schmelzpunkt: 316°C
¹H NMR (CDCl₃, 400 MHz): d= 2.80 (s, 3H, Methyl-H), 5.17 (s, 2H, Methylen-H), ), 5.18 (s, 1H, Hydroxy-H), 6.14, 6.17 (s, je 1H, Brückenköpfe), 7.02-7.04 (m, 4H, Phenyl-H), 7.45-7.47 (m, 2H, Phenyl-H), 7.51-7.53 (m, 4H, Phenyl-H), 7.95-7.98 (m, 1H, Naphtyl-H), 8.15-8.17 (m, 1H, Naphtyl-H)
IR: v/cm⁻¹ = 3700-3300, 3000-3100, 2980, 1450, 760.

### Beispiel 9a:

### Synthese von 6-Chlormethyl-11-methyl-5,12-dihydro-5,12-o-benzeno-naphthacen

Analoge Durchführung der Reaktion zu Beispiel 9, mit dem Unterschied, daß die Reaktionszeit 48 Stunden bei Raumtemperatur und anschließend weitere 5 Stunden am Rückfluß betrugt. Danach wurde der farblose Feststoff abgetrennt, mit Diethylether und halbkonzentrierter Salzsäure nachgewaschen, in Chloroform gelöst, bis zur Entfernung der Violettfärbung mit 5n HCl gewaschen, die organische Phase abgetrennt, getrocknet und einrotiert. Es ergaben sich 12g (83%) farbloses Rohprodukt. Der Feststoff wurde erneut in der Siedehitze in CH₂Cl₂ gelöst und mit Hexan kristallisiert. Man erhielt 10g (76%) reines, farbloses 6-Chlormethyl-11-methyl-5,12-dihydro-5,12-o-benzeno-naphthacen vom Schmelzpunkt 308-312°C.

### Beispiel 10: Synthese von 6-Formyl-11-methyl-5, 12-dihydro-5, 12-o-benzeno-naphthacen (6-Carboxy-11-naphtotriptycen)

Zu einer Lösung von 10g (28.7mmol) 6-Hydroxymethyl-11-methyl-5, 12-dihydro-6,11-o-benzeno-naphthacen und 2.85g (7.0mmol) Tetrabutylammoniumhydrogensulfat in 350ml CH₂Cl₂ wurden unter Rühren bei 0°C eine Lösung von 2.85g (7.0mmol) Natriumdichromat-dihydrat in 60 ml Wasser und 30 ml Schwefelsäure (ca. 3molar) langsam zugetropft. Nach Beendigung der Zugabe wurde eine weitere Stunde bei Raumtemperatur gerührt.
Es wurde in einen Scheidetrichter überführt und die Phasen getrennt. Die Wasserphase wurde dreimal mit Methylenchlorid (50ml) extrahiert. Die vereinigten org. Phasen wurden 2 mal mit 50ml aq. NaCl-Lösung und einmal mit reinem Wasser gewaschen und anschließend über Natriumsulfat getrocknet. Nach Einrotieren wurden 10g (100%) Rohprodukt isoliert.
Das Rohprodukt wurde in Dimethoxyethan gelöst und in der Siedehitze mit Wasser gefällt. Man erhielt 9.0g (90%) annähernd farbloses 6-Formyl-11-methyl-5,12-dihydro-5,12-o-benzeno-naphthacen vom Schmelzpunkt 246-248°C.

### Beispiel

Danach filtrierte man vom entstandenen Braunstein ab, wusch mit wenig 2N NaOH und 2 x mit 10 ml Methylenchlorid nach. Man trennte die Phasen und extrahiert die organische Phase 3x mit 20 ml 2N NaOH. Die basische Wasserphase wurde 2 x mit 20 ml Diethylether gewaschen. Danach wurde solange halbkonzentrierte HCl zugetropft, bis ein pH-Wert von etwa 2-3 erreicht wurde. Bei pH 6 begann die Säure aus der Lösung auszufallen. Die Wasserphase wurde dann 3 x mit 30 ml Essigester extrahiert, die organische Phase wurde getrocknet und auf etwa 30 ml eingeengt. Nach Erwärmen auf etwa 60 °C wurde dann Hexan zugesetzt, bis ein weißer Niederschlag nur sich noch langsam auflöste. Man kühlte im Kühlschrank und saugte den ausgefallenen kristallinen Niederschlag ab. Man erhielt 300 mg (14%) Naphtotriptycen-6,11-dicarbonsäure.
Schmelzpunkt: 310-316°C
¹H NMR (DMSO-*d*₆, 400 MHz): d = 5.88 (s, 2H, Brückenkopf-H), 7.08-7.11 (m, 4H, Phenyl-H), 7.48-7.50 (m, 4H, 4 Phenyl-H), 7.57-7.60 (m, 2H, Naphtyl-H), 7.88-7.91 (m, 2H, Naphthyl-H), 13.96 (s, 2H, Carboxyl-H)

### Beispiel B8: Synthese von 5,12-Bis-(5-p-tert-butylphenyl-1,3,4-oxdiazol-2-yl)-6,11-dihydro-6,11-o-benzeno-naphtacen

1.0 g (2.55 mmol) Naphtotriptycen-6,11-dicarbonsäure wurde bei Raumtemperatur in einem 200 ml Zweihalskolben unter Feuchtigkeitsausschluß mit 2 ml trockenem N,N-Dimethylformamid suspendiert und mit 10 ml frisch dest. Thionylchlorid versetzt. Die sofort einsetzende Gasentwicklung war nach 15 min abgeklungen. Die Lösung wurde dann noch für weitere 3 h am Rückfluß gekocht. N,N-Dimethylformamid und überschüssiges Thionylchlorid wurden im Vakuum abdestilliert und der Rückstand wurde noch dreimal mit jeweils 30 ml Petrolether (90°C) aufgerührt und es wurde erneut im Vakuum abdestilliert. Das erhaltene gelblich gefärbte Säurechlorid wurde dann in 30 ml Pyridin (getrocknet über Molsieb) gelöst und mit einer Lösung von 1.1g (5.30 mmol) *p*-*tert*.-Butylphenyltetrazol in 20 ml Pyridin versetzt und anschließend 2h am Rückfluß gekocht. Die abgekühlte Lösung wurde in 300 ml Wasser eingegossen und der ausgefallene Niederschlag abgesaugt und getrocknet.

Das erhaltene Rohprodukt wurde in Chloroform gelöst und zweimal mit je 100 ml 2N HCl extrahiert, und mit 100ml Wasser gewaschen. Die organische Phase wurde über Na₂SO₄ getrocknet und einrotiert. Nach Chromatographie mit Hexan/EE 1:1 über Kieselgel wurden 600 mg farbloses, stark blau fluoreszierendes 5,12-Bis-(5-*p-tert* butylphenyl-1,3,4-oxdiazol-2-yl)-6,11-dihydro-6,11-o-benzeno-naphtacen erhalten.
Schmelzpunkt 285°C-288°C.
¹H-NMR (400 MHz; DMSO-*d*₆): δ =1.38 (s, 18H, *t*-Butyl-H), 6.15 (s, 2H, Brückenkopf-H), 7.13-7.15 (m, 4H, Phenyl-H), 7.55-7.58 (m, 4H, Phenyl-H), 7.63-7.65 (m, 2H, Naphthyl-H), 7.75-7.78 (m, 4H, Phenyl-H) 8.04-8.06 m, 2H, Naphthyl-H)), 8.15-8.18 (m, 4H, Phenyl-H).

### Beispiel 13: Synthese von 6-Brommethyl-11-chlormethylnaphtotriptycen

1.0 g (2.7 mmol) 6-Methyl-11-chlormethylnaphtotriptycen wurde in 150 ml trockenem Tetrachlorkohlenstoff suspendiert und zusammen mit 0.5 g (2.7 mmol) N-Bromsuccinimid, sowie 0.08 g (0.5 mmol) Diazoisobutyronitril (AZIBN) unter Bestrahlung mit einer 500 W Halogenlampe zum gelinden Rückfluß erhitzt. Nach 3 Stunden ließ man auf Raumtemperatur erkalten und trennte durch Filtration von dem ausgefallenen Succinimid ab. Es wurde auf 60 ml eingeengt und bei 50°C durch Zugabe von Hexan kristallisiert. Nach Kühlen im Eisfach wurde der Niederschlag abgesaugt und getrocknet. Nach Umkristallisation aus CCl₄/Hexan wurde 440 mg (44%) 6-Brommethyl-11-chlormethylnaphtotriptycen als farblose Kristalle erhalten.
Schmelzpunkt: 291-295°C
¹H NMR (CDCl₃, 400 MHz): d= 5.19 (s, 2H, Brommethyl-H), 5.28 (s, 2H, Chlormethyl-H), 5.95 (d, 2H, Brückenkopf-H), 7.06-7.10 (m, 4H, Phenyl-H), 7.49-7.47 (m, 6H, 4 Phenyl-H, 2 Naphthyl-H), 8.01-8.03 (m, 2H, Naphthyl-H).

### Beispiel 14: Synthese von 1-Formyl-4-methyl-naphtotriptycen

10 g (28.7 mmol) 6-Hydroxymethyl-11-methylnaphtotriptycen und 1.0 g (2.9 mmol) Tetrabutylammoniumhydrogensulfat wurden in 200 ml CH₂Cl₂ gelöst und unter Rühren bei 0°C wurde eine Lösung von 2.85 g (7.0 mmol) Natriumdichromat-dihydrat in einer Mischung aus 60 ml Wasser und 30 ml Schwefelsäure (= 6 M) langsam zugetropft. Nach Beendigung der Zugabe wurde weitere drei Stunden bei Raumtemperatur gerührt.
Nach Phasentrennung wurde die Wasserphase dreimal mit je Methylenchlorid (50ml) extrahiert. Die vereinigten organischen Phasen wurden 2 x mit je 50ml Wasser gewaschen und anschließend über Natriumsulfat getrocknet. Nach Einrotieren verblieben 10.0g (100%) eines gelbgrünen Feststoffs.
Das Rohprodukt wurde aus Eisessig umkristallisiert. Man erhielt nach Trocknung 9.7 g (98%) 1-Formyl-4-methylnaphtotriptycen als gelbgrünes Pulver. Schmelzpunkt: 246-248°C
¹H NMR (DMSO-*d*₆, 400 MHz): d = 2.91 (s, 3H, Methyl-H) 6.30 und 6.90 (s, je 1H, Brückenkopf-H), 6.90-7.10 (m, 4H, Phenyl-H), 7.55-7.59 (m, 6H, 4 Phenyl-H, 2 Naphthyl-H), 8.07-8.09 (m, 1H, Naphthyl-H), 8.92-8.94 (m, 1H, Naphthyl-H), 11.25 (s, 1 H, Aldehyd-H)

### Beispiel 15: Synthese von 6,11-Diformylnaphtotriptycen

2.0g (4.49 mmol) 6-Brommethyl-11-chlormethylnaphthotriptycen wurde zusammen mit 3.0 g Natriumhydrogencarbonat in 30 ml DMSO suspendiert und drei Stunden bei einer Temperatur von 75-80°C gehalten. Anschließend wurde die Reaktionsmischung in Wasser eingegossen und der ausgefallene beige-gelbe Niederschlag abgesaugt. Nach Trocknung wurde in möglichst wenig Eisessig in der Siedehitze gelöst und durch Abkühlung wurde langsam kristallisiert. Nach Trocknung erhielt man 0.60 g (1.67 mmol, 37%) 6,11-Diformylnaphtotriptycen als leicht gelbe Kristalle.
¹H NMR (DMSO-*d*₆, 400 MHz): d= 6.80 (s, 2H, Brückenkopf-H), 7.11-7.13 (m, 4H, Phenyl-H), 7.59-7.61 (m, 4H, Phenyl-H) 7.65-7.67(m, 2H, Naphthyl-H), 8.71-8.74 (m, 2H, Naphthyl-H), 11.26 (s, 2 H, Formyl-H).

### Beispiel 16: Synthese von 2,3,5,6-Bis(9,10-Dihydro-9,10-anthracenyl)-1,4-cyclohexadion

In einem 500 ml Dreihalskolben wurden bei Eiskühlung unter N₂ 10.0 g (56 mmol) Anthracen und 3.02 g (28 mmol) *p*-Benzochinon (frisch sublimiert) in 150 ml getrocknetem Methylenchlorid suspendiert. Anschließend fügte man 3.72 g (28 mmol) AlCl₃ zu. Dabei verfärbte sich die Reaktionslösung von anfänglich rötlich nach tiefblau. Nach erfolgter Zugabe wurde das Eisbad entfernt und man rührte bei Raumtemperatur noch etwa 2,5 Stunden weiter.
Nach dieser Zeit wurden erneut 100 ml CH₂Cl₂ hinzugefügt und die Reaktionslösung auf Eis gegossen. Dabei fiel ein ockerfarbener Niederschlag aus, der im Methylenchlorid bei Raumtemperatur, in Lösung wieder in Lösung ging. Die Phasen wurden in einem Scheidetrichter getrennt, die Wasserphase zweimal mit 25 ml CH₂Cl₂ extrahiert und die vereinigten Methylenchloridextrakte getrocknet. Nach Abziehen des Lösemittels verblieb 7.36 g (56 %) Rohprodukt als ein bräunlichbeiger Rückstand.
Zur Reinigung wurde in 75 ml Xylol refluxiert. Man ließ erkalten und saugte den Niederschlag ab.
Weitere Umkristallisation aus Dioxan lieferte 6.04 g (46 %) weißes Produkt. Schmelzpunkt: 247°C

### Beispiel 17: Synthese von Dihydrobistriptycenchinon

2.20 g (7.7 mmol) Triptycenchinon und 1.38 g (7.7 mmol) Anthracen wurden in 100 ml *p*-Xylol in einem 250 ml Kolben unter Schutzgas suspendiert. Beim Aufheizen auf Siedetemperatur entstand eine dunkelgelb gefärbte Lösung, aus der nach etwa 30 min gelbe Kristalle auszufallen begannen. Nach etwa 4 Stunden ließ man auf Raumtemperatur abkühlen. Der Niederschlag wurde abgesaugt, mit Xylol nachgewaschen und getrocknet.
Es wurden 3.20 g (90 %) Rohprodukt erhalten (Schmelzpunkt: 298°C)
Die Substanz wurde etwa 20 min in *p*-Xylol refluxiert. Nach Erkalten und Absaugen erhielt man 2.9 g (82 %) Dihydrobistriptycenchinon als gelbliche Kristalle. Schmelzpunkt: >345°C
¹H NMR (CDCl₃, 400 MHz): d= 3.07 (m, 2H, alkyl-H), 4.70 (s, 2H, Brücknkopf-H), 5.53 (s, 2H, Brückenkopf-H), 6.35-6.39 und 6.81-6.85 (m, AB-System, zus. 4H, Phenyl-H), 6.91-6.97 (m, 4H, Phenyl-H), 7.13-7.19 (m, 4H, Phenyl-H), 7.32-7.38 (m, 4H, Phenyl-H).

### Beispiel 18: Synthese von Bistriptycenhydrochinon

Unter N₂ wurden 2.06 g (4.5 mmol) Dihydrobistriptycenchinon in 50 ml Eisessig unter Zugabe von 1.5 ml 48%ige wäßrige Bromwasserstoffsäure suspendiert und auf Rückflußtemperatur erwärmt. Nach insgesamt 4 h Rückfluß ließ man erkalten, saugte den Niederschlag ab und wusch mit Wasser neutral. Das Bistriptycenhydrochinon wurde unter Sauerstoff- und Lichtausschluß getrocknet und aufbewahrt; man erhielt 1.74 g (84 %) Rohprodukt.
Die erhaltene Substanz wurde unter N₂ in *iso*-Propanol umkristallisiert. Es wurden 1.44 g (70 %) Bistriptycenhydrochinon als farblose kristalline Substanz erhalten.

### Schmelzpunkt: 270°C (Zersetzung)

### Synthese von CN-Trp-BND

### Beispiel 19:

### Synthese von 6-Cyano-11-methyl-5,12-Dihydro-5,12-o-benzeno-naphthacen

3g (8,7mmol) 6-Formyl-11-methyl-5,12-dihydro-5,12-o-benzeno-naphthacen , 0.5g (2.6mmol) p-Toluolsulfonsäure, 0.7g (10.0mmol) Hydroxylammoniumchlorid und 5.0g (41.0mmol) wasserfreies Magnesiumsulfat wurden in 50ml p-Xylol gelöst und sechs Stunden auf 130°C erhitzt. Danach wurde die Reaktionslösung auf 50°C abgekühlt, mit 30 ml Chloroform versetzt und noch leicht warm durch ein Faltenfilter filtriert. Das abgetrennte Magnesiumsulfat wurde mit Chloroform nachgewaschen und verworfen. Das Filtrat wurde einrotiert und der so erhaltene Feststoff, in 30ml Essigsäureanhydrid 4 Stunden bei 120°C gehalten. Danach wurde abgekühlt, wobei langsam rohes, leicht bräunliches, blau fluoreszierendes Produkt auskristallisierte. Der Niederschlag wurde abgesaugt und mit wenig Eisessig nachgewaschen. Das Filtrat wurde erneut auf 100°C erhitzt und sehr vorsichtig in kleinen Portionen mit 25ml Wasser zur teilweisen Hydrolyse des Acetanhydrids versetzt. Beim Abkühlen fiel weiteres Produkt aus, das ebenfalls abgesaugt wurde. Nach Umkristallisation der vereinigten Feststoffe aus Xylol wurden 2.2g (76%) farbloses 6-Cyano-11-methyl-5,12-dihydro-5,12-o-benzeno-naphthacen mit einem Schmelzpunkt oberhalb von 370°C erhalten.
¹H-NMR; 400 MHz/DMSO:
δ/ppm = 2.91 (s, 3H) 6.0 und 6.3 (s, je 1H), 7.10-7.12 (m, 4H), 7.59-7.68 (m, 6H, 4-Phenyl, 2-Naphthyl-H), 7.93-7.96(m, 1H), 8.06-8.08 (m, 1H);
¹H-NMR; 400 MHz/CDCl₃:
δ/ppm = 2.68 (s, 3H) 5.9 und 6.0(s, je 1H), 7.05-7.10 (m, 4H), 7.41-7.56 (m, 6H),7.92-7.94 (m, 1H), 8.06 (m, 1H);
IR(KBr): v/cm⁻¹: 3042, 2950, 2212, 1580, 1462, 1197, 1158, 765, 578;

### Beispiel 20:

### Synthese von 6-Cyano-11-brommethyl-5,12-dihydro-5,12-o-benzeno-naphthacen

2. 1 g (5.8mmol) 6-Cyano-11-methyl-6,11-dihydro-6,11-o-benzeno-naphthacen wurden unter Stickstoffüberlagerung in einem 250ml Zweihalskolben in 200ml trockenem Tetrachlorkohlenstoff bei 90°C durch Bestrahlung mit einer Quecksilberlampe zum gelinden Rückfluß erhitzt. 1.24g (7 mmol) NBS und 50mg (0.3mmol) Diazoisobutyronitril wurden homogen vermischt. Jeweils 0.25g dieser Mischung wurden nach und nach im Abstand von 30 Minuten zur Reaktionslösung gegeben. Nach Beendigung der Zugabe wurde weitere zwei Stunden refluxiert. Danach ließ man zuerst auf Raumtemperatur erkalten und trennte vom unlöslichen Succinimid ab. Die Tetrachlorkohlenstofflösung wurde einrotiert, der erhaltene Feststoff in möglichst wenig Chloroform gelöst und in der Siedehitze mit 100ml Hexan gefällt. Nach Kühlen im Eisfach wurde der Niederschlag abgesaugt und getrocknet. Man erhielt 2.2g (90%) farbloses Pulver von 6-Cyano-11-Brommethyl-5,12-dihydro-5,12-o-benzeno-naphthacen vom Schmelzpunkt 308-315°C.
¹H-NMR: 400 MHz/CDCl₃:
δ/ppm = 5.14 (s, 2H), 5.93 und 6.0 (je s, je 1H), 7.08-7.13 (m, 4H, ), 7.51-7.57 (m,
4H), 7.59-7.63 (m, 2H), 8.00-8.02 (m, 1H), 8.1-8.14 (m, 1H);
IR(KBr): v/cm⁻¹: 3069, 2970, 2850, 2216, 1512, 1462, 1207, 1158, 762, 575;

### Beispiel 21:

### Synthese von 6-Cyano-11-tosylmethyl-5,12-dihydro-5,12-o-benzeno-naphthacen

In einem 250ml Dreihalskolben wurden bei Raumtemperatur 1.0g (2.4mmol) 6-cyano-11-Brommethyl-5,12-dihydro-5,12-o-benzeno-naphthacen in 150ml CH₃CN suspendiert. Dazu wurde eine Lösung von 0.73g (2.6mmol) Silbertosylat in 20ml CH₃CN getropft. Die Suspension wurde zwei Stunden bei 70°C Danach ließ man abkühlen, trennte vom gelblichen Silberbromid ab und rotiert das Acetonitril ein. Es verblieb eine relativ viskose Substanz, die durch Zugabe von 3ml Ether kristallisiert wurde. Es wurde abgesaugt mit wenig Diethylether nachgewaschen und getrocknet. Es ergeben sich 1.0g (80%) kristallines 6-Cyano-11-tosylmethyl-5,12-dihydro-5,12-o-benzeno-naphthacen.
¹H-NMR; 400MHz/CDCl₃:
δ/ppm = 2.28, (s, 3H), 5.96, (s, 2H), 6.02 u. 6.03, (je s, je 1H), 6.73-6.75 (m, 2H), 7.13-7.20, ( m, 6H), 7.52-7.61, (m, 6H), 7.89-7.91, (m, 1H), 8.01-8.03, (m, 1H);

### Beispiel 22:

### Synthese von 6-Cyano-11-formyl-5,12-dihydro-5,12-o-benzeno-naphthacen

Das erhaltene, nicht weiter gereinigte Rohprodukt aus der Reaktion zu Beispiel 21 wurde anschließend zusammen mit 2g NaHCO₃ in 20ml abs. DMSO auf 100°C aufgeheizt (5 Minuten). Nach dieser Zeit war die Reaktion beendet und wurde durch Eingießen in 200ml Wasser abgebrochen. Der dabei ausfallende Aldehyd wurde abgesaugt und getrocknet. Man erhielt 0.7g (65%)annähernd farbloses 6-Cyano-11-formyl-5,12-dihydro-5,12-o-benzeno-naphthacen.
¹H-NMR; 400MHz/DMSO:
δ/ppm = 6.16 u. 6.89, (je s, je 1H), 7.14-7.15 (m, 4H, ), 7.63-7.68 (m, 4H), 7.74-7.78 (m, 2H), 8.06-8.08 (m, 1H), 8.80-8.82 (m, 1H), 11.25 (s, 1H);
IR: ν/cm⁻¹: 3069, 3024, 2915, 2222, 1690, 1462, 1195, 1158, 1048, 762, 599;

### Beispiel 23:

### Synthese von 6-Cyano-11-carboxyl-5,12-dihydro-5,12-o-benzeno-naphthacen

Zu einer Suspension aus 1.0g (2.8mmol) 6-Cyano-11-formyl-5,12-dihydro-5,12-obenzeno-naphthacen, 5ml H₂O₂, 15ml wässr. Pufferlösung und 100ml Acetonitril wurde bei Raumtemperatur eine Lösung von 130mg Natriumchlorit in 20ml Wasser getropft. Dabei entsteht eine gelbliche Suspension, deren Gelbfärbung im Verlauf der Reaktion verschwindet. Die Suspension wurde vier Stunden bei Raumtemperatur gerührt, wobei letztlich eine gelbe Lösung resultiert.
Es wurden 30ml Wasser hinzugefügt und das Acetonitril einrotiert, wodurch die Säure auskristallisiert. Nach Absaugen wurde die noch feuchte Carbonsäure in 100ml 2n NaOH gelöst. Die trübe Suspension wurde durch einen Faltenfilter filtriert und die klare basische Lösung mit konz. HCl angesäuert, woraufhin die Säure leicht gelb gefärbt ausfällt.

Sie wurde abgesaugt und in verdünnter 2n Natronlauge erneut gelöst. Die basische Lösung wurde einmal mit einer Mischung aus 30ml Ether extrahiert, von der org. Phase abgetrennte und unter kräftigem Rühren mit konz. HCl versetzt bis ein pH-Wert von 3 bestehen bleibt. Nach Erkalten und Absaugen erhielt man 700mg (68%) 6-Cyano-11-carboxyl-5,12-dihydro-5,12-o-benzeno-naphthacen vom Schmelzpunkt 346-350°C.
¹H-NMR; 400 MHz/DMSO:
δ/ppm = 5.93 und 6.13, (je s, je 1H), 7.11-7.16 (m, 4H), 7.54-7.56 (m, 2H), 7.66-7.77 (m, 4H), 7.95-7.97 (m, 1H) 8.05-8.07, (m, 1H), 14.4 (s, 1H);
IR(KBr): v/cm⁻¹: 3400-2700, 2222, 1735, 1693, 1510, 1461, 1196, 763, 747;

### Beispiel 24:

### Synthese von 2,5-Bis-((11'-cyano-5',12'-dihydro-5',12'-o-benzeno-naphthacen)-6'-yl-)-1,3,4-oxadiazol

4.7g (12.6mmol) 6-Cyano-11-carboxyl-5,12-dihydro-5,12-o-benzeno-naphthacen wurden in einem 250ml Zweihalskolben unter Feuchtigkeitsausschluß mit 3ml abs. Dimethylformamid suspendiert und mit 60ml frisch dest. Thionylchlorid versetzt. Die sofort einsetzende Entwicklung von SO₂ und HCl war nach 15 Minuten weitestgehend abgeklungen und die Lösung wurde für 4h am Rückfluß gekocht. DMF und überschüssiges Thionylchlorid wurden anschließend im Vakuum abdestilliert und der Rückstand nochmals mit 50ml Petrolether (90°C) aufgerührt und erneut scharf abdestilliert (Vakuum). Es wurden weitere 50ml Petrolether hinzugefügt und das Säurechlorid unter N₂ mittels einer Schlenk-Apparatur abgesaugt und nur wenig getrocknet. Man erhielt 7.4g (150%) annähernd farbloses, noch feuchtes Carbonsäurechlorid.
Die Hälfte des erhaltenen annähernd farblosen Säurechlorids wurden in 100ml abs. Dioxan gelöst und bei Raumtemperatur langsam zu einer Lösung von 2.0ml Hydrazinhydrat (80%) in 25ml Dioxan getropft. Nach beendeter Zugabe wurde noch 1 Stunde weitergerührt. Die Reaktionsmischung wurde anschließend in 500ml Wasser eingegossen und das ausgefallene Produkt wurde abgesaugt und getrocknet. Man erhielt 2.02g (82%) farbloses Carbonsäurehydrazid, das ohne Reinigung weiter umgesetzt wurde.
Das Carbonsäurehydrazid wurde unter Feuchtigkeitsauschluß in einer Mischung aus 50ml abs. NEt₃ und 50ml Dioxan gelöst und unter kräftigem Rühren bei Raumtemperatur zu einer Lösung der zurückbehaltenen, zweiten Hälfte des Säurechlorids in 120ml abs. Dioxan getropft. Nach beendeter Zugabe wurde 2 Stunden refluxiert, wobei sich die Lösung zunehmend dunkler färbte. Die noch warme Lösung wurde in 200ml Wasser eingegossen und kurz gerührt. Der ausgefallene Niederschlag wurde abgesaugt, mit reichlich Wasser nachgewaschen und getrocknet. Man erhielt leicht bräunliches Diaroythydrazin das ebenfalls ohne weitere Reinigung weiter umgesetzt wurde.

4.5g des rohen N,N'-Dinaphthoyltriptycenhydrazin wurden in 200ml frisch dest. Phosphoroxychlorid acht Stunden refluxiert. Danach wurden etwa 150ml POCl₃ abdestilliert und die verbleibende Lösung in 300ml 2n NaOH eingetropft. Es wurde mit 2N HCl auf pH6-7 eingestellt. Der ausgefallene Niederschlag wurde abgesaugt und getrocknet. (3.7g = 88%)
Das Rohprodukt wurde in 350ml DME gelöst und 30 Minuten mit Aktivkohle refluxiert. Danach wurde durch einen Faltenfilter filtriert.
Die verbleibende Lösung wurde zur Trockne einrotiert. Der weißlich beige Feststoff wurde in NMP aufgenommen und bei 70°C mit MeOH kristallisiert. Durch weitere zwei Kristallisationen aus Dioxan/MeOH erhielt man 450mg farbloses 2,5-Bis-((11'-cyano-5',12'-dihydro-5',12'-o-benzeno-naphthacen)-6'-yl-)-1,3,4-oxadiazol, das nicht unzersetzt schmelzbar war.
T_{g} = 165°C;
¹H NMR; 400 MHz/CDCl₃):
δ/ppm = 6.16 u. 6.20 (2 x s, je 2H), 7.08-7.17 (m, 8H), 7.43-7.44 (m, 4H, ), 7.71-7.63 (m, 4+2H),7.69-7.73 (m, 2H), 8.09-8.11 (m, 2H), 8.226-8.28 (m, 2H);
Masse: FD, M/Z(%): 701.9 (100%), [M⁺];
UV-VIS: Absorption: λₘₐₓ= 340nm;
Fluoreszenz (Lösung): (Lösung 8E-6mol/l), 411 nm (ε=45000);
Fluoreszenz (Film): Spincoating, Chlorbenzol, 10mg/ml, 1000U/Min: 428nm;
IR(KBr): v/cm⁻¹: 3069, 2959, 2850, 2223, 1550, 1506, 1460, 1156, 1121, 756, 577;
Cyclovoltammetrie:
Reduktion; 0.1M TBAHFP/THF, (Fc/Fc⁺), 100mV/s: E¹_{1/2} = -1.939mV, E²_{1/2} = -2108mV;

### Synthese von (t-BuPD)₂NTrp

### Beispiel 25:

### Synthese von 6,11-Diformyl-5, 12-dihydro-5, 12-o-benzeno-naphthacen

Zu einer Suspension aus 4.70g (10.6mmol) 6-Brommethyl-11-chlormethyl-5,12-dihydro-5,12-o-benzeno-naphthacen in 300ml Acetonitril wurden in einem 500ml Zweihalskolben 5.9g (23mmol) p-Toluolsulfonsäure-Silbersalz in 50ml Acetonitril getropft und unter Lichtausschluß über Nacht bei Raumtemperatur gerührt. Danach wurde vom ausgefallenen Silberbromid abgetrennt und das Acetonitril einrotiert. Die letzten 10 ml Lösung wurden mit 10ml Et₂O versetzt, woraufhin Kristallisation des Bistosylats einsetzt. Nach Kühlung wurde abgesaugt. Man erhielt 4.2g (70%) farbloses 6, 11-Bis-(tosylmethyl)-5,12-dihydro-5,12-o-benzeno-naphthacen das in 70ml getrocknetem DMSO suspendiert und zusammen mit 5g NaHCO₃ während 15 Minuten auf 100°C aufgeheizt wurde. Anschließend wurde abgekühlt und die noch etwa 50°C warme Lösung in 300ml Wasser eingegossen. Der ausgefallene Niederschlag wurde abgesaugt und getrocknet. Es ergaben sich 2.1g (70%) gelblich-beiges Rohprodukt. Nach Umkristallisation aus 80%iger Essigsäure wurden 1.7g (65%) gelblich kristallines 6,11-Diformyl-5,12-dihydro-5,12-o-benzeno-naphthacen vom Schmelzpunkt 272-277°C erhalten.
¹H-NMR: (400 MHz/CDCl₃):
δ/ppm =2.39 (s, 6H), 5.26 (s, 4H), 5.66 (s, 2H), 6.69 (s, 2H), 6.98-7.00 (m, 4H), 7.17-7.19 (m, 4H), 7.33-7.35 (m, 4H), 7.65-7.67 (m, 4H);
IR(KBr): v/cm⁻¹: 3069, 3020, 2870, 1685, 1462, 1213, 1160, 981, 753, 633, 553;

### Beispiel 26:

### Synthese von 5,12-Dihydro-5,12-o-benzeno-naphthacen-6,11-dicarbonsäure

2.41g (6.7mmol) 6,11-Diformyl-5,12-dihydro-5,12-o-benzeno-naphthacen wurden in einem 1 L Zweihalskolben in einem Gemisch aus 200ml Acetonitril, 10ml 30%igem Wasserstoffperoxid und 20ml Pufferlösung (pH 3) bei Raumtemperatur suspendiert. Über einen Tropftrichter wurden 1.70g (19mmol) Natriumchlorit (80%) in 10ml Wasser zugetropft. Nach zwei Stunden Reaktionszeit wurde die entstandene gelbliche Lösung mit weiteren 30ml Wasser versetzt und das Acetonitril abrotiert. Der sich bildende Niederschlag wurde nach Kühlung abgesaugt und mit wenig Wasser nachgewaschen.
Die noch feuchte Säure wurde in 100ml 3N NaOH gelöst und einmal mit 30ml Diethylether extrahiert. Die basische Lösung wurde bis zum vollständigen Ausfallen der Säure nach Kühlung im Eisbad mit konz. HCl versetzt bis pH 3 bestehen bleibt. Der Niederschlag wurde abgesaugt und sorgfältig getrocknet. Es ergaben sich 2.5g (96%) farblos kristalline 5,12-Dihydro-5,12-o-benzeno-naphthacen-6,11-dicarbonsäure vom Schmelzpunkt 318-323°C.
¹H NMR; (400 MHz/DMSO):
δ =5.88 (s, 2H), 7.08-7.11 (m, 4H, ), 7.48-7.50 (m, 4H), 7.57-7.60 (m, 2H), 7.88-7.91 (m, 2H), 13.96 (s, 2H);
IR: v/cm⁻¹: 3424, 3400-2650, 2641, 1695, 1460, 1242, 1208, 766, 566;

### Beispiel 27:

### Synthese von 5,12-Dihydro-5,12-o-benzeno-naphthacen-6,11-dicarbonsäure-dichlorid

2.4g (6mmol) 5,12-Dihydro-5,12-o-benzeno-naphthacen-6,11-dicarbonsäure wurden unter Feuchtigkeitsausschluß in einem 100ml Rundkolben mit 1.5ml abs. DMF suspendiert und bei Raumtemperatur anschließend mit 50ml Thionylchlorid versetzt. Die sofort einsetzende Gasentwicklung war nach 15 Minuten weitgehend abgeklungen woraufhin acht Stunden zum Rückfluß erhitzt wurde. Danach wurde das Thionylchlorid abdestilliert und 3x mit 30ml Petrolether versetzt und erneut abdestilliert. Danach wurde mit 50 Petrolether versetzt und unter Feuchtigkeitsausschluß abgesaugt. Man erhielt 2.3g (96%) annähernd farbloses 5,12-Dihydro-5,12-o-benzeno-naphthacen-6,11-dicarbonsäuredichlorid.

### Beispiel 28:

### Synthese von 6, 11-Bis-(5'-(p-tert. butylphenyl)-1,3,4-oxadiazol)-2'yl)-5,12-dihydro-5,12-o-benzeno-naphthacen-(tBuPD)₂NTrp

1.0g (2.55mmol) 5,12-Dihydro-5,12-o-benzeno-naphthacen-6,11-dicarbonsäure wurden in einem 200ml Zweihalskolben unter Feuchtigkeitsausschluß, bei Raumtemperatur, mit 2ml abs. Dimethylformamid suspendiert und mit 10ml frisch dest. Thionylchlorid versetzt. Die sofort einsetzende Entwicklung von SO₂ und HCl war nach 15 Minuten weitestgehend abgeklungen, woraufhin die Lösung für 3 Stunden am Rückfluß gekocht wurde. DMF und überschüssiges Thionylchlorid wurden im Vakuum abdestilliert und der Rückstand noch dreimal mit jeweils 30 ml Petrolether (Sdp.90°C) aufgerührt und erneut abdestilliert. Das erhaltene gelblich gefärbte Säurechlorid wurde nach Absaugen unter Feuchtigkeitsausschluß dann in 30ml abs. Pyridin gelöst und mit einer Lösung von 1.1g (5.30mmol) *p*-tert.-Butylphenyltetrazol in 20ml Pyridin versetzt und anschließend 2h am Rückfluß gekocht. Die abgekühlte Lösung wurde in 300ml Wasser eingegossen und der ausgefallene Niederschlag abgesaugt und getrocknet. Das erhaltene Rohprodukt wurde in Chloroform gelöst und zweimal mit je 100mt 2N HCl extrahiert, und mit 100ml Wasser gewaschen. Die org. Phase wurde über Na₂SO₄ getrocknet und einrotiert. Nach Chromatographie mit Hexan/EE 1:1 über Kieselgel wurden 600mg farbloses, stark blau fluoreszierendes 6,11-Bis-(5'-(p-tert, butylphenyl-1,3,4-oxadiazol)-2yl)-5,12-dihydro-5,12-o-benzeno-naphthacen vom Schmelzpunkt 285°C-288°C (T_{g} = 165°C) erhalten.
¹H-NMR; 400 MHz/DMSO:
δ =1.38 (s, 18H) 6.15 (s, 2H), 7.13-7.15 (m, 4H), 7.55-7.58 (m, 4H), 7.63-7.65 (m, 2H) , 7.75-7.78 (m, 4H), 8.04-8.06 (m, 2H), 8.15-8.18 (m, 4H);
IR(KBr): ν/cm⁻¹: 3070-3050, 2960-2868, 1614, 1548, 1495, 1192, 1188, 842, 763;
Masse: FD, 8kV: m/z: 704.1 (100%) [M⁺];
UV-VIS: Absorption: λₘₐₓ= 318nm (ε = 50000);
Fluoreszenz (Lösung): (CH₂Cl₂: 10⁻⁶ mol/l), 433nm;
Fluoreszenz (Film): Spincoating, Chlorbenzol, [1000U/min], 10mg/ml, λ_{Em} = 423nm;
Cyclovoltammetrie:
Reduktion; 0.1 M TBAHFP/THF, (Fc/Fc⁺), 100mV/s: E¹_{1/2} = -2140mV,
E²_{1/2} = -2333mV(irrev.);

## Patentansprüche

1. Verwendung von Triptycenderivaten der Formel (I) in Elektrolumineszenzvorrichtungen, 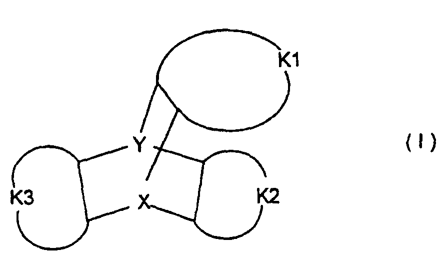 wobei die Symbole in der Formel folgende Bedeutungen haben:
K¹,K²,K³ sind, gleich oder verschieden, mono- oder polycyclische Systeme, die gegebenenfalls Heteroatome enthalten und die gegebenenfalls substituiert sind;
X, Y sind, gleich oder verschieden, CR¹, N, P, As, SiR²;
R¹ ist, gleich oder verschieden, H, Halogen, Pseudohalogen oder ein Kohlenwasserstoffrest mit 1 bis 30 C-Atomen, der gegebenenfalls auch Heteroatome enthält.
R² ist, gleich oder verschieden, ein Kohlenwasserstoffrest mit 1 bis 30 C-Atomen, der gegebenenfalls auch Heteroatome enthält.

2. Verwendung gemäß Anspruch 1, **dadurch gekennzeichnet, daß** mindestens eine der Gruppen K¹⁻³ in der Formel (I) ein Fluorophor ist.

3. Verwendung gemäß Anspruch 1, **dadurch gekennzeichnet, daß** die Gruppen K¹⁻³ konjugierte Systeme sind.

4. Verwendung gemäß einem oder mehreren der Ansprüche 1 bis 3, **dadurch gekennzeichnet, daß** das Triptycenderivat eine Verbindung der Formel (II) oder Formel (III) ist, 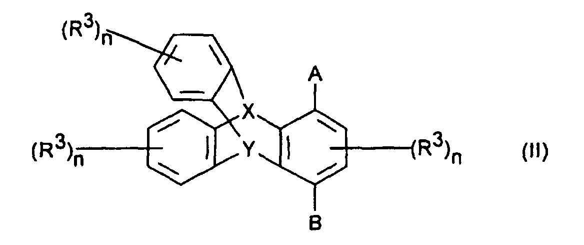 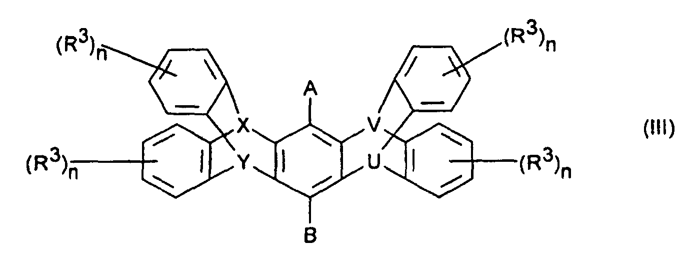 wobei die Symbole und Indizes folgende Bedeutungen haben:
X, Y, U, V sind, gleich oder verschieden, CR¹, N, P, As, SiR²;
R¹ ist, gleich oder verschieden, H, Halogen, Pseudohalogen oder ein Kohlenwasserstoffrest mit 1 bis 30 C-Atomen, der gegebenenfalls auch Heteroatome enthält;
R² ist, gleich oder verschieden, ein Kohlenwasserstoffrest mit 1 bis 30 C-Atomen, der gegebenenfalls auch Heteroatome enthält;
R³ ist, gleich oder verschieden, F, Cl, Br, I, CN, NO₂, eine unverzweigte oder verzweigte Alkylgruppe mit 1 bis 22 C-Atomen, wobei eine oder mehrere -CH₂-Gruppen durch -O-, -S-, -SO₃-, -O-CO-, -CO-O-, Aryl oder Heteroaryl (mit jeweils 4 bis 10 C-Atomen) ersetzt sein können, mit der Maßgabe, daß nicht zwei Sauerstoffatome unmittelbar miteinander gebunden sein dürfen, und wobei ein, mehrere oder alle H-Atome durch F ersetzt sein können, und wobei zwei am selben Ring befindliche Substituenten R³ unter Ausbildung eines Ringes oder weiteren anellierten Ringsystems miteinander verknüpft sein können oder auch, gegebenenfalls partiell, hydriert sein können und Substituenten tragen können, mit der Maßgabe, daß die Zahl der Substituenten nicht größer ist als die Gesamtzahl der C-Atome;
n ist, gleich oder verschieden, 0,1,2,3,4,5;
A, B sind, gleich oder verschieden, Gruppen der Formel
(-M)ₐ(-E)_{b}(-M)_{c}-(-E)_{d}(-M)ₑ(-E)_{f}(-M)_{g}(-E)ₕ-R⁴
wobei die Symbole und Indizes folgende Bedeutungen haben:
M ist, gleich oder verschieden -CR⁵=CR⁶, -C≡C-, -CR⁷=N-, -N=CR⁷-;
E ist, gleich oder verschieden, Pyrazin-2,5-diyl, Pyridazin-3,6-diyl, Pyridin-2,5-diyl, Pyrimidin-2,5-diyl, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Naphthalin-2,6-diyl, Naphthalin-1,4-diyl oder Naphthalin-1,5-diyl, wobei eine oder zwei CH-Gruppen durch N ersetzt sein können, 1,3-Oxazol-2,4-diyl, 1,3-Oxazol-2,5-diyl, (1,3,4)-Oxadiazol-2,5-diyl, 4,4'-Biphenylen, Anthracen-diyl, Carbazol-diyl, Benzoxazol-diyl, Inden-2,5-diyl, Inden-2,6-diyl, wobei in den Ringsystemen ein oder mehrere H-Atome durch Reste R⁸ substituiert sein können;
R⁴,R⁵,R⁶,R⁷ sind gleich oder verschieden
a) Wasserstoff, -F, -Cl, -CF₃, -CN, NR⁹R¹⁰
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O- oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können;
R⁸ ist gleich oder verschieden
a) -F, -Cl, -CF₃, -CN, NO₂-
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O-, -NH-, N(C₁-C₁₀-Alkyl), -N-Phenyl-, -N-Tolyl-, -N(C₂H₅-OCH₃)- oder -Si(CH₃)₂- ersetzt sein können und/oder
b2) eine oder mehrere CH₂-Gruppen durch -CH=CH-, -C≡C-, 1,4-Phenylen ersetzt sein können und/oder
b3) ein oder mehrere H-Atome durch F, CN und/oder Cl ersetzt sein können;
R⁹, R¹⁰ sind gleich oder verschieden
a) Wasserstoff
b) ein geradkettiger oder verzweigter Alkylrest (mit oder ohne asymmetrisches C-Atom) mit 1 bis 20 C-Atomen, wobei
b1) eine oder mehrere nicht untereinander oder dem Stickstoff benachbarte CH₂-Gruppen durch -O-, -S-, -CO-, -CO-O-, -O-CO-, -O-CO-O-, oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können und
b4) R⁸ und R⁹ zusammen auch einen Ring bilden können;
a, b, c, d, e, f, g, h sind unabhängig voneinander 0 oder 1.

5. Verwendung gemäß Anspruch 4, **dadurch gekennzeichnet, daß** die Summe der Indizes a-h in der Formel (II) oder (III) mindestens 1 beträgt.

6. Verwendung gemäß Anspruch 5, **dadurch gekennzeichnet, daß** man ein Triptycenderivat der Formel(IV), (V) oder (VI) einsetzt, 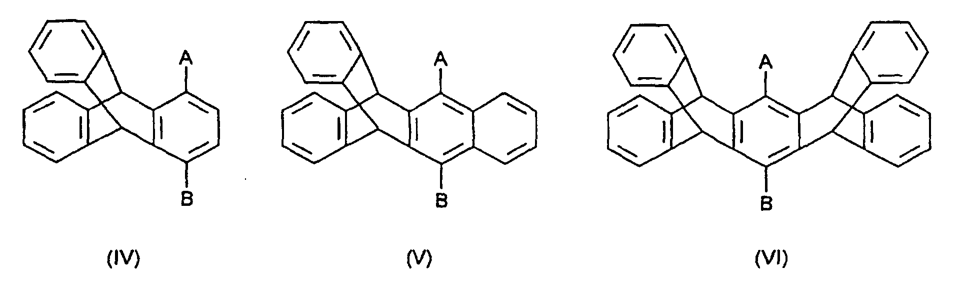 wobei die Gruppen A und B die gleichen Bedeutungen wie in den Formeln (II)/(III) in Anspruch 4 haben.

7. Verwendung gemäß Anspruch 6, **gekennzeichnet durch** ein Triptycenderivat aus der Gruppe (IV), (V), (VI) a-i: 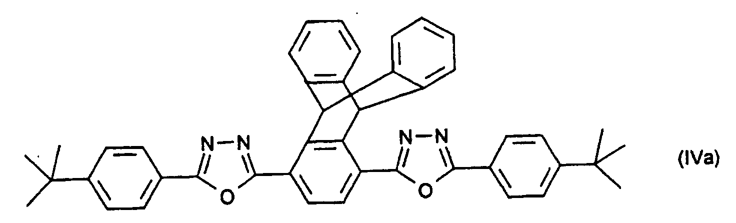 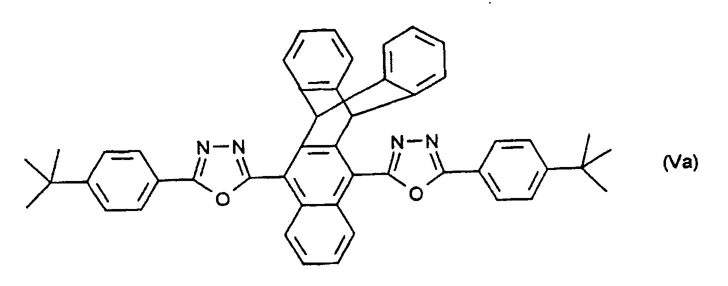 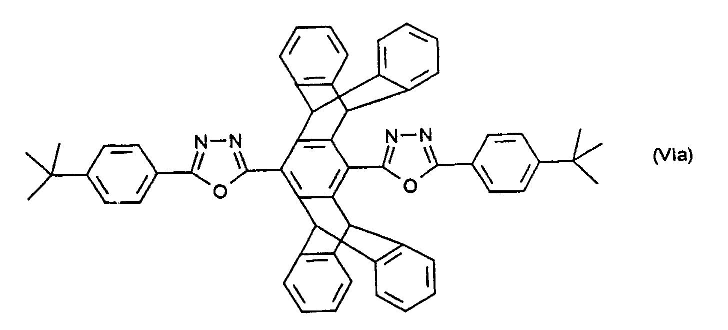 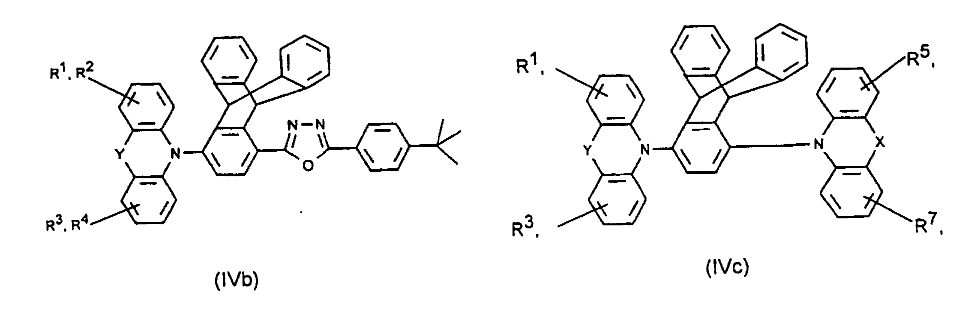 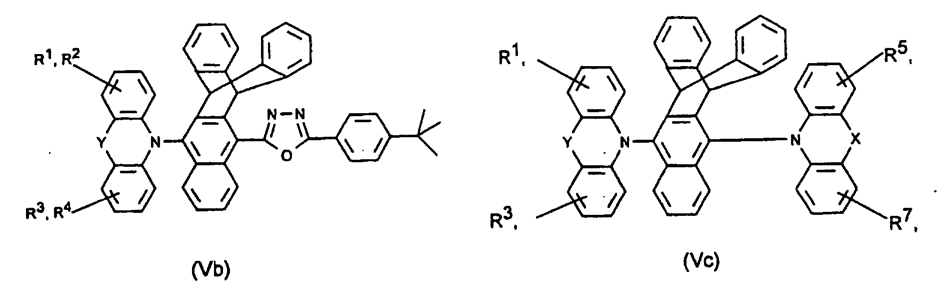 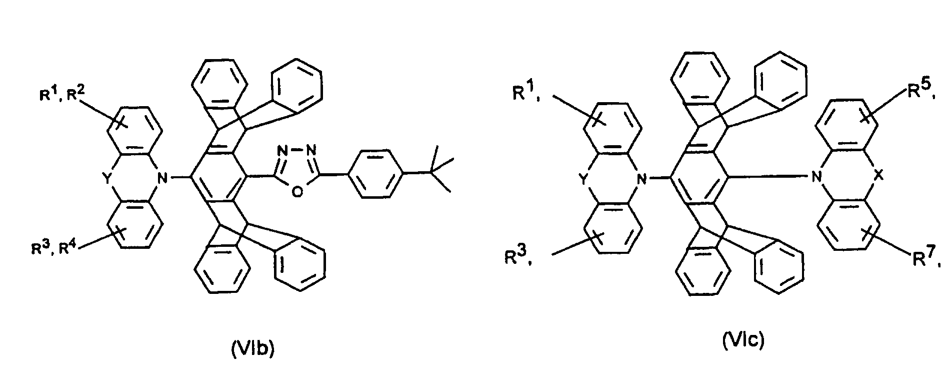 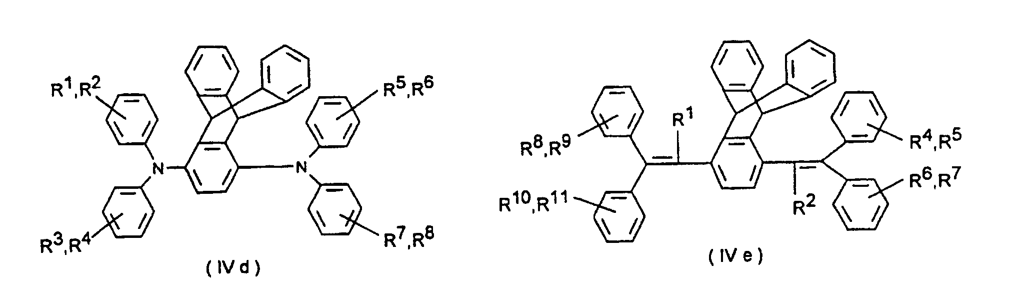 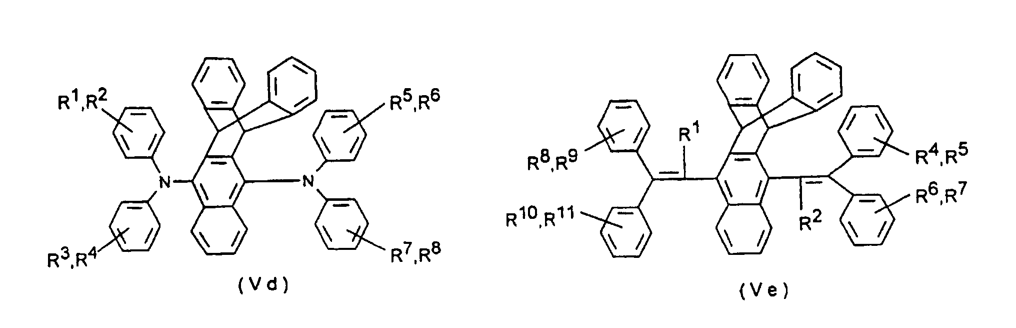 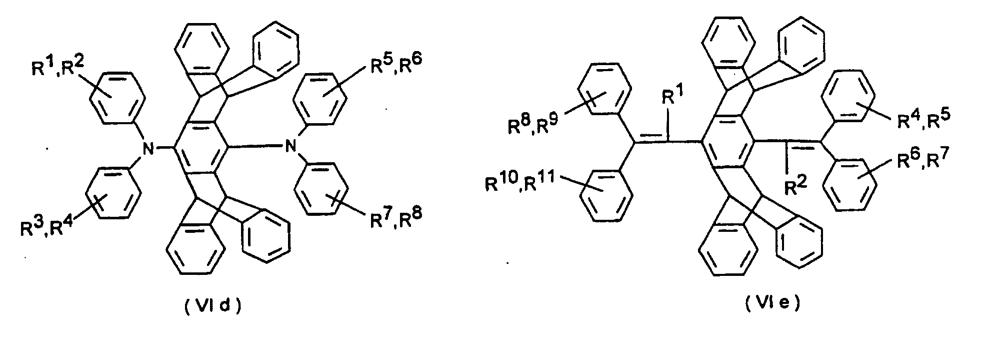 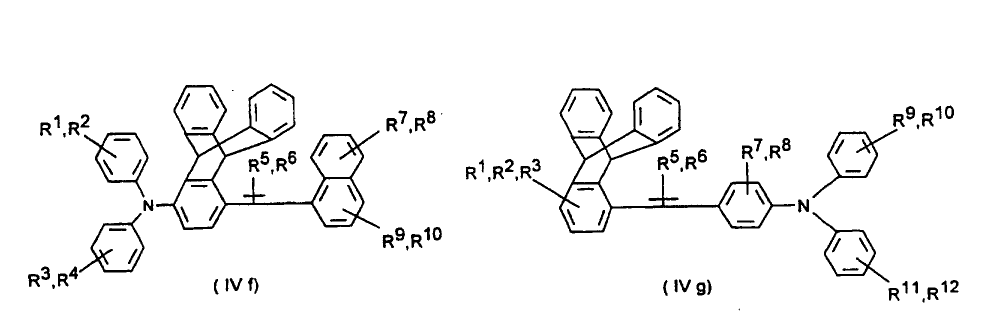 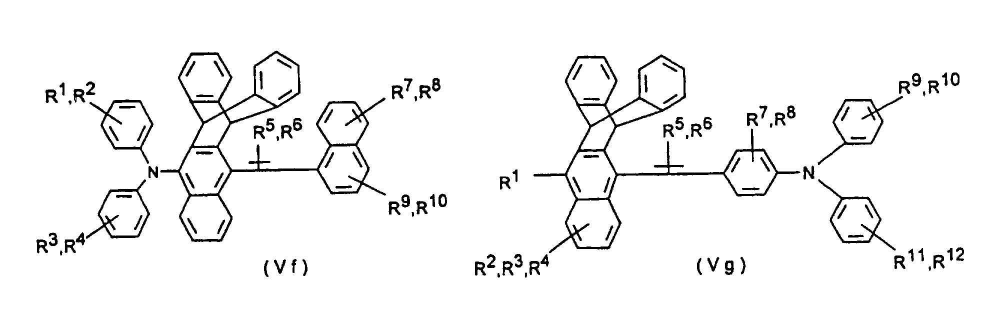 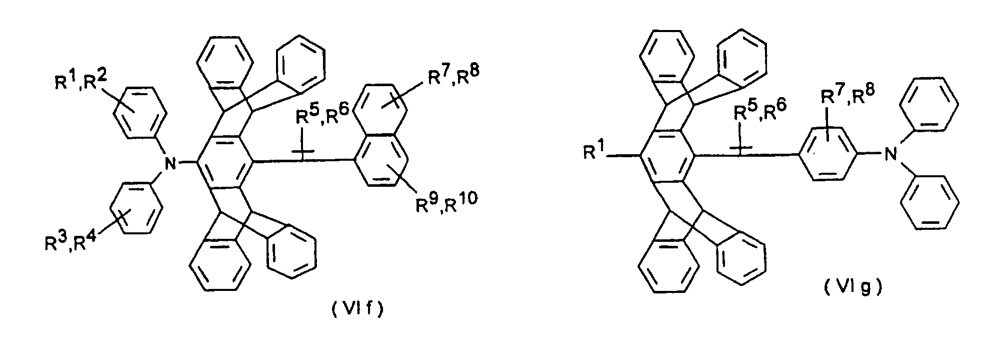 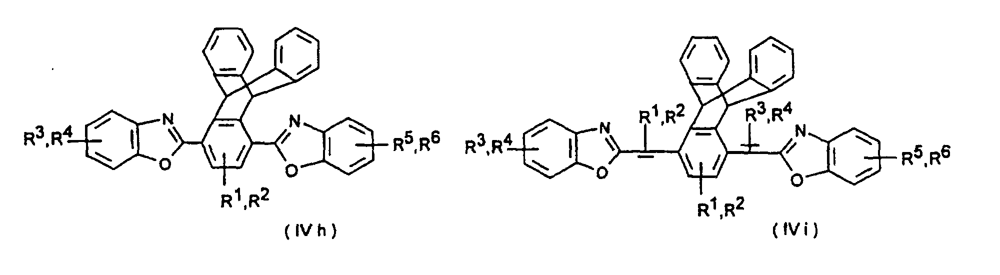 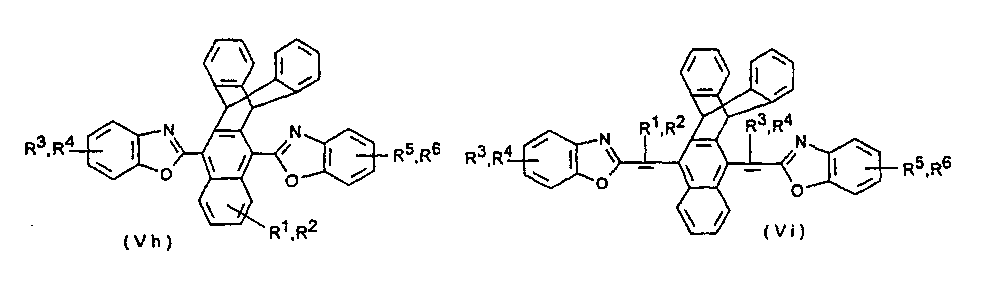 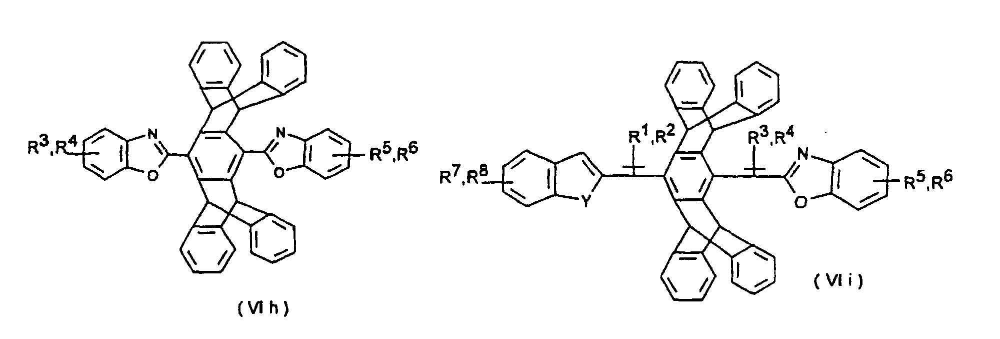 wobei die Symbole folgende Bedeutungen haben:
Y ist -O-, -S-, -NR¹¹-, -CR¹⁰R¹¹- ;
R¹⁻¹¹ sind, gleich oder verschieden F, Cl, Br, I, CN, NO₂, eine unverzweigte oder verzweigte Alkylgruppe mit 1 bis 22 C-Atomen, wobei eine oder mehrere -CH₂ Gruppen **durch** -O-, -S-, -SO₃-, -O-CO-, -CO-O-, Aryl oder Heteroaryl (mit jeweils 4 bis 10 C-Atomen) ersetzt sein können, mit der Maßgabe, daß nicht zwei Sauerstoffatome unmittelbar

8. Triptycenderivat der Formel (II) 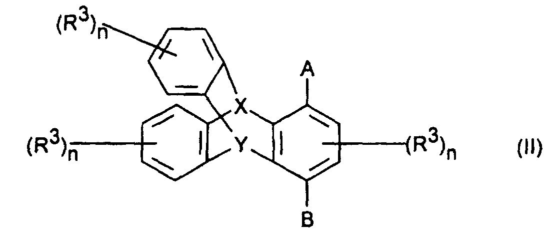 wobei die Symbole und Indizes folgende Bedeutungen haben:
X, Y sind, gleich oder verschieden, CR¹, N, P, As, SiR²;
R¹ ist, gleich oder verschieden, H, Halogen, Pseudohalogen oder ein Kohlenwasserstoffrest mit 1 bis 30 C-Atomen, der gegebenenfalls auch Heteroatome enthält;
R² ist, gleich oder verschieden, ein Kohlenwasserstoffrest mit 1 bis 30 C-Atomen, der gegebenenfalls auch Heteroatome enthält;
R³ ist, gleich oder verschieden, F, Cl, Br, I, CN, NO₂, eine unverzweigte oder verzweigte Alkylgruppe mit 1 bis 22 C-Atomen, wobei eine oder mehrere -CH₂-Gruppen durch -O-, -S-, -SO₃-, -O-CO-, -CO-O-, Aryl oder Heteroaryl (mit jeweils 4 bis 10 C-Atomen) ersetzt sein können, mit der Maßgabe, daß nicht zwei Sauerstoffatome unmittelbar miteinander gebunden sein dürfen, und wobei ein, mehrere oder alle H-Atome durch F ersetzt sein können, und wobei zwei am selben Ring befindliche Substituenten R³ unter Ausbildung eines Ringes oder weiteren anellierten Ringsystems miteinander verknüpft sein können oder auch, gegebenenfalls partiell, hydriert sein können und Substituenten tragen können, mit der Maßgabe, daß die Zahl der Substituenten nicht größer ist als die Gesamtzahl der C-Atome;
n ist, gleich oder verschieden, 0,1,2,3,4,5;
A, B sind, gleich oder verschieden, Gruppen der Formel
(-M)ₐ(-E)_{b}(-M)_{c}-(-E)_{d}(-M)ₑ(-E)_{f}(-M)_{g}(-E)ₕ-R⁴
wobei die Symbole und Indizes folgende Bedeutungen haben:
M ist, gleich oder verschieden -CR⁵=CR⁶, -C≡C-, -CR⁷=N-, -N=CR⁷-;
E ist, gleich oder verschieden, Pyrazin-2,5-diyl, Pyridazin-3,6-diyl, Pyridin-2,5-diyl, Pyrimidin-2,5-diyl, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Naphthalin-2,6-diyl, Naphthalin-1,4-diyl oder Naphthalin-1,5-diyl, wobei eine oder zwei CH-Gruppen durch N ersetzt sein können, 1,3-Oxazol-2,4-diyl, 1,3-Oxazol-2,5-diyl, (1,3,4)-Oxadiazol-2,5-diyl, 4,4'-Biphenylen, Anthracen-diyl, Garbazol-diyl, Benzoxazol-diyl, Inden-2,5-diyl, Inden-2,6-diyl, wobei in den Ringsystemen ein oder mehrere H-Atome durch Reste R⁸ substituiert sein können;
R⁵,R⁶,R⁷ sind gleich oder verschieden
a) Wasserstoff, -F, -Cl, -CF₃, -CN, NR⁹R¹⁰
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O- oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können;
R⁴ sind gleich oder verschieden
a) -F, -Cl, -CF₃, -CN, NR⁹R¹⁰
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O- oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können;
R⁸ ist gleich oder verschieden
a) -F, -Cl, -CF₃, -CN, NO₂-
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O-, -NH-, N(C₁-C₁₀-Alkyl), -N-Phenyl-, -N-Tolyl-, -N(C₂H₅-OCH₃)- oder -Si(CH₃)₂- ersetzt sein können und/oder
b2) eine oder mehrere CH₂-Gruppen durch -CH=CH-, -C≡C-, 1,4-Phenylen ersetzt sein können und/oder
b3) ein oder mehrere H-Atome durch F, CN und/oder Cl ersetzt sein können;
R⁹, R¹⁰ sind gleich oder verschieden
a) Wasserstoff
b) ein geradkettiger oder verzweigter Alkylrest (mit oder ohne asymmetrisches C-Atom) mit 1 bis 20 C-Atomen, wobei
b1) eine oder mehrere nicht untereinander oder dem Stickstoff benachbarte CH₂-Gruppen durch -O-, -S-, -CO-, -CO-O-, -O-CO-, -O-CO-O-, oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können und
b4) R⁸ und R⁹ zusammen auch einen Ring bilden können;
a, b, c, d, e, f, g, h sind unabhängig voneinander 0 oder 1.

9. Triptycenderivate der Formel III 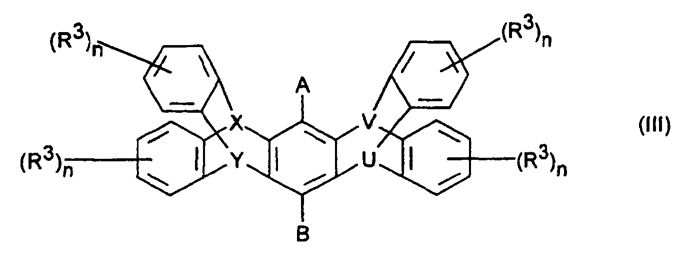 wobei die Symbole und Indizes folgende Bedeutungen haben:
X, Y, U, V sind, gleich oder verschieden, CR¹, N, P, As, SiR²;
R¹ ist, gleich oder verschieden, H, Halogen, Pseudohalogen oder ein Kohlenwasserstoffrest mit 1 bis 30 C-Atomen, der gegebenenfalls auch Heteroatome enthält;
R² ist, gleich oder verschieden, ein Kohlenwasserstoffrest mit 1 bis 30 C-Atomen, der gegebenenfalls auch Heteroatome enthält;
R³ ist, gleich oder verschieden, F, Cl, Br, I, CN, NO₂, eine unverzweigte oder verzweigte Alkylgruppe mit 1 bis 22 C-Atomen, wobei eine oder mehrere -CH₂-Gruppen durch -O-, -S-, -SO₃-, -O-CO-, -CO-O-, Aryl oder Heteroaryl (mit jeweils 4 bis 10 C-Atomen) ersetzt sein können, mit der Maßgabe, daß nicht zwei Sauerstoffatome unmittelbar miteinander gebunden sein dürfen, und wobei ein, mehrere oder alle H-Atome durch F ersetzt sein können, und wobei zwei am selben Ring befindliche Substituenten R² unter Ausbildung eines Ringes oder weiteren anellierten Ringsystems miteinander verknüpft sein können oder auch, gegebenenfalls partiell, hydriert sein können und Substituenten tragen können, mit der Maßgabe, daß die Zahl der Substituenten nicht größer ist als die Gesamtzahl der C-Atome;
n ist, gleich oder verschieden, 0,1,2,3,4,5;
A, B sind, gleich oder verschieden, Gruppen der Formel
(-M)ₐ(-E)_{b}(-M)_{c}-(-E)_{d}(-M)ₑ(-E)_{f}(-M)_{g}(-E)ₕ-R⁴
wobei die Symbole und Indizes folgende Bedeutungen haben:
M ist, gleich oder verschieden -CR⁵=CR⁶, -C≡C-, -CR⁷=N-, -N=CR⁷-;
E ist, gleich oder verschieden, Pyrazin-2,5-diyl, Pyridazin-3,6-diyl, Pyridin-2,5-diyl, Pyrimidin-2,5-diyl, (1,3,4)-Thiadiazol-2,5-diyl, 1,3-Thiazol-2,4-diyl, 1,3-Thiazol-2,5-diyl, Thiophen-2,4-diyl, Thiophen-2,5-diyl, Naphthalin-2,6-diyl, Naphthalin-1,4-diyl oder Naphthalin-1,5-diyl, wobei eine oder zwei CH-Gruppen durch N ersetzt sein können, 1,3-Oxazol-2,4-diyl, 1,3-Oxazol-2,5-diyl, (1,3,4)-Oxadiazol-2,5-diyl, 4,4'-Biphenylen, Anthracen-diyl, Carbazol-diyl, Benzoxazol-diyl, Inden-2,5-diyl, Inden-2,6-diyl, wobei in den Ringsystemen ein oder mehrere H-Atome durch Reste R⁸ substituiert sein können;
R⁴,R⁵,R⁶,R⁷ sind gleich oder verschieden
a) Wasserstoff, -F, -Cl, -CF₃, -CN, NR⁹R¹⁰
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O- oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können;
R⁸ ist gleich oder verschieden
a) -F, -Cl, -CF₃, -CN, NO₂-
b) ein geradkettiger oder verzweigter Alkylrest (mit oder ohne asymmetrisches C-Atom) mit 1 bis 20 C-Atomen, wobei
b1) eine oder mehrere nicht benachbarte und nicht terminale CH₂-Gruppen durch -O-, -S-, -CO-, -CO-O-, -O-CO-, -O-CO-O-, -NH-, N(C₁-C₁₀-Alkyl), -N-Phenyl-, -N-Tolyl-, -N(C₂H₅-OCH₃)- oder -Si(CH₃)₂- ersetzt sein können und/oder
b2) eine oder mehrere CH₂-Gruppen durch -CH=CH-, -C≡C-, 1,4-Phenylen ersetzt sein können und/oder
b3) ein oder mehrere H-Atome durch F, CN und/oder Cl ersetzt sein können;
R⁹, R¹⁰ sind gleich oder verschieden
a) Wasserstoff
b) ein geradkettiger oder verzweigter Alkylrest (mit oder ohne asymmetrisches C-Atom) mit 1 bis 20 C-Atomen, wobei
b1) eine oder mehrere nicht untereinander oder dem Stickstoff benachbarte CH₂-Gruppen durch -O-, -S-, -CO-, -CO-O-, -O-CO-, -O-CO-O-, oder -Si(CH₃)₂- ersetzt sein können und/oder
b2) eine oder mehrere CH₂-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
b3) ein oder mehrere H-Atome durch F, CN und/oder Cl ersetzt sein können und
b4) R⁸ und R⁹ zusammen auch einen Ring bilden können;
a, b, c, d, e, f, g, h sind unabhängig voneinander 0 oder 1.
miteinander gebunden sein dürfen, und wobei ein, mehrere oder alle H-Atome durch F ersetzt sein können, und wobei zwei am selben Ring befindliche Substituenten R² unter Ausbildung eines Ringes oder weiteren anellierten Ringsystems miteinander verknüpft sein können oder auch, gegebenenfalls partiell, hydriert sein können und Substituenten tragen können, mit der Maßgabe, daß die Zahl der Substituenten nicht größer ist als die Gesamtzahl der C-Atome.

10. Elektrolumineszenzvorrichtung, enthaltend eine oder mehrere aktive Schichten, **dadurch gekennzeichnet, daß** mindestens eine aktive Schicht ein oder mehrere Triptycenderivate gemäß einem oder mehreren der Ansprüche 1 bis 6 enthält.

## Claims

1. Use of triptycene derivatives having the formula (I) in electro-luminescent devices, 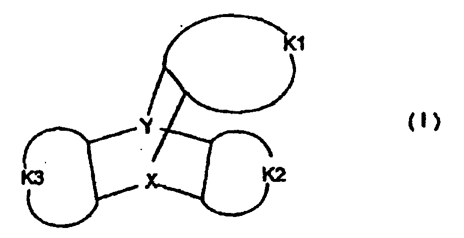 where the symbols appearing in the formula have the following meanings:
K¹, K², K³, are any one or a selection from mono- or polycyclic systems, which may contain hetero-atoms and which may be substituted;
X, Y, are any one or a selection from CR¹, N, P, As, SiR²;
R¹ is any one of or a selection from H, halogen, pseudo-halogen or a hydrocarbon residue with 1 to 30 C-atoms, which may also contain heteroatoms.
R² is any one or a selection of hydrocarbon residue(s) with 1 to 30 C-atoms which may also contain hetero-atoms.

2. Use in accordance with claim 1, **characterised in that** at least one of the groups K¹⁻³ in Formula (I) is a fluorphore.

3. Use in accordance with claim 1, **characterised in that** the groups K¹⁻³ are conjugate systems.

4. Use in accordance with one or more of claims 1 to 3, **characterised in that** the triptycene derivative is a compound having the formula (II) or (III), 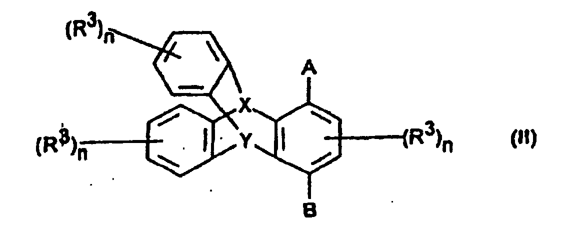 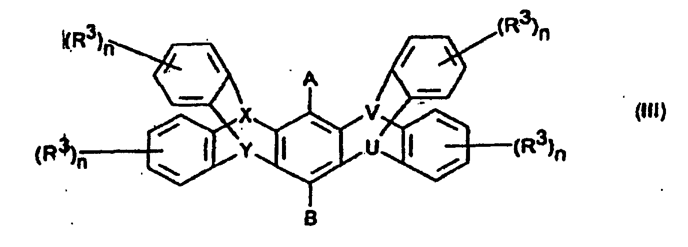 where the symbols and indices have the following meanings:
X, Y, U, V are any one of or a selection from CR¹, N, P, As, SiR²;
R¹ is any one of or a selection from H, halogen, pseudo-halogen or a hydrocarbon residue having 1 to 30 C-atoms, which may also contain hetero-atoms;
R² is any one or a selection of hydrocarbon residue(s) with 1 to 30 C-atoms, which may also contain hetero-atoms;
R³ is any one of or a selection from F, Cl, Br, I, CN, NO₂, an unbranched or branched alky group with 1 to 22 C-atoms, where one or more of the -CH₂- groups may be substituted with -O-, -S-, -SO₃-, -O-CO-, -CO-O-, an aryl or heteroaryl group (with respectively 4 to 10 C-atoms) subject the requirement that no two oxygen atoms may be directly bonded to one another and where one, several or all H-atoms may be replaced by F and where two substituents R³ located in the same ring may be linked together so as to form a ring or other anellated ring system or which, furthermore, may be hydrated, possibly only partially, and can carry substituents, subject to the requirement that the number of the substituents is not greater than the total number of C-atoms;
n is, individually or collectively, 0,1, 2, 3 , 4 5;
A, B are any one or a selection of group(s) of the formula
(-M)ₐ(-E)_{b}(-M)_{c}-(-E)_{d}(-M)ₑ(-E)_{f}(-M)_{g}(-E)ₕ-R⁴
where the symbols and indices have the following meanings:
M is any one of or a selection from -CR⁵=CR⁶, -C≡C-, -CR⁷=N-, -N=CR⁷-,
E is any one of or a selection from pyrazine-2,5-diyl, pyridazine-3,6-diyl, pyridine-2,5-diyl, pyrimidine-2,5-diyl, (1, 3, 4)-thiadiazole-2,5-diyl, 1,3-thiazole-2,4-diyl, 1,3-thiazole-2,5-diyl, thiophene-2,4-diyl, thiophene-2,5-diyl, naphthalene-2,6-diyl, naphthalene-1,4-diyl or naphthalene-1,5-diyl where one or two CH-groups may be replaced by N, 1,3-oxazole-2,4-diyl, 1,3-oxazole-2,5-diyl, (1,3,4)-oxadizole-2,5-diyl, 4,4'-biphenylene, anthracerie-diyl, carbazole-diyl, benzoxazole-diyl, indene-2,5-diyl, indene-2,6-diyl, where in the ring systems one or more H-atoms may be substituted with R⁸ residues;
R⁴, R⁵, R⁶, R7 are any one of or a selection from
a) hydrogen, -F, -Cl, -CF₃, -CN, NR⁹R¹⁰
b) a straight-chain or a branched-chain alkyl residue (with or without an asymmetrical C-atom) with I to 20 C-atoms, where
b1) one or more non-adjacent and non-terminal CH₂- groups may be replaced with -O-, -S-, -CO-, -CO-O-, -O-CO-, -O-CO-O- or Si(CH₃)₂ and / or
b2) one or more CH₂-groups may be replaced by -CH=CH-, -C≡C-, cyclopropane-1,2-diyl, 1,4-phenylene, 1,4-cyclohexylene or 1,3-cyclopentylene and / or
b3) one or more H-atoms may be replaced by F, CN and / or Cl
R⁸ is any one of or a selection from
a) -F, -Cl, -CF₃, -CN, NO₂-
b) a straight-chain or a branched-chain alkyl residue (with or without an asymmetrical C-atom) with 1 to 20 C-atoms, where
b1) one or more non-adjacent and non-terminal CH₂- groups may be replaced with -O-, -S-, -CO-, -CO-O-, -O-CO-, -O-CO-O-, -NH-, N(C₁-C₁₀-alkyl), -N-phenyl-, -N-tolyl-, -N(CH₂H₅-OCH₃)- or -Si(CH₃)₂- and / or
b2) one or more CH₂-groups may be replaced by -CH=CH-, -C≡C-, 1,4-phenylene and / or
b3) one or more H-atoms may be replaced by F, CN and / or Cl
R⁹, R¹⁰ are any one of or a selection from
a) hydrogen
b) a straight-chain or a branched alkyl residue (with or without an asymmetric C-atom), with 1 to 20 C-atoms, where
b1) one or more CH₂-groups not adjacent to one another or the nitrogen may be replaced by -O-, -S-, -CO-, -CO-O-, -O-CO-, -O-CO-O- or -Si(CH₃)₂- and / or
b2) one or more CH₂ groups may be replaced by -CH=CH-, -C≡C-, cyclopropane-1,2-diyl, 1,4-phenylene, 1,4-cyclohexylene or 1,3-cyclopentylene and / or
b3) one or more H-atoms may be replaced by F, CN and / or Cl and
b4) R⁸ and R⁹ can together form a ring;
a, b, c, d, e, f, g, and h independently of one another are 0 or 1.

5. Use in accordance with claim 4, **characterised in that** the sum of the indices a-h in Formula (II) or Formula (III) amounts to a minimum of 1.

6. Use in accordance with claim 5, **characterised in that** a triptycene derivative in accordance with Formula (IV), (V) or (VI) is used 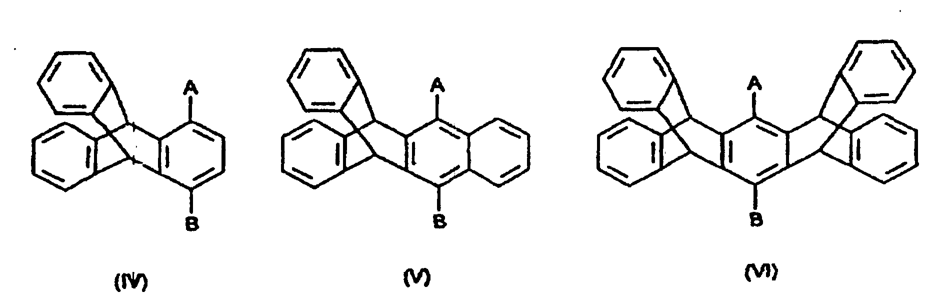 where the groups A and B have the same significance as in Formulae (II) and (III) in claim 4.

7. Use in accordance with claim 6, **characterised by** a triptycene derivative selected from the Groups (IV), (V) or (VI) a - i: 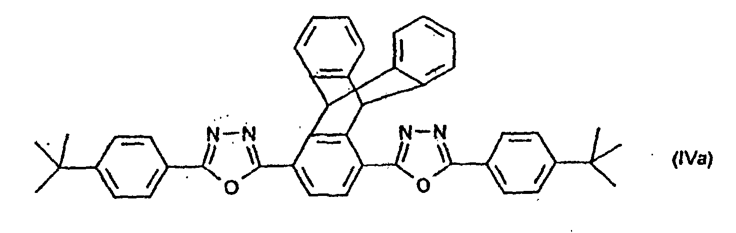 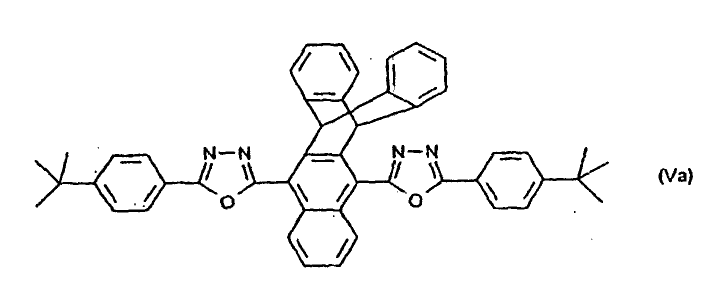 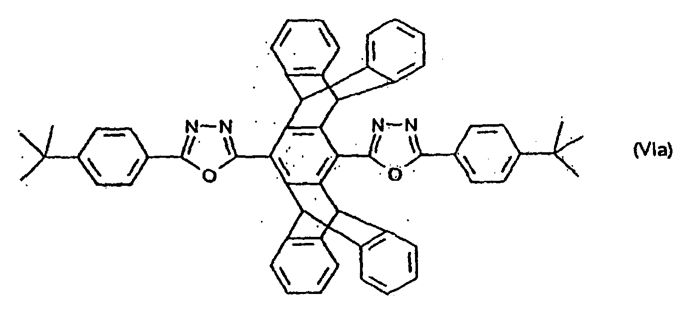 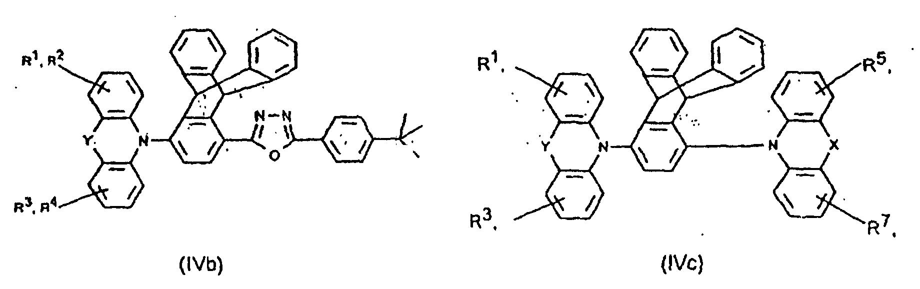 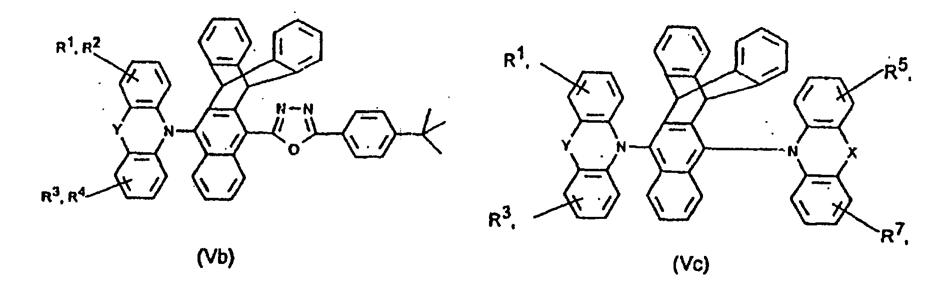 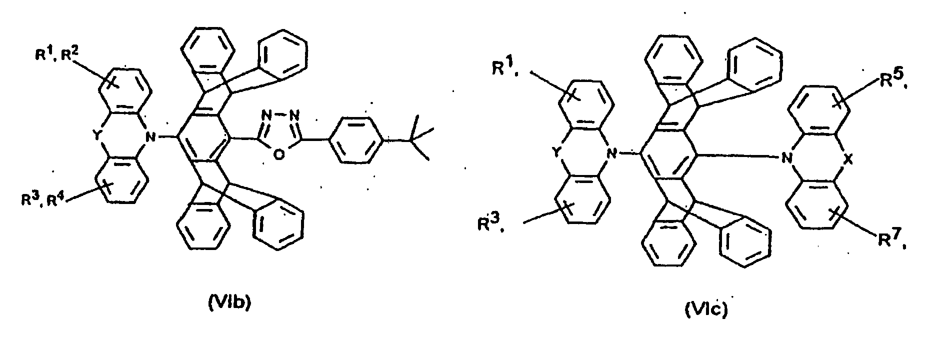 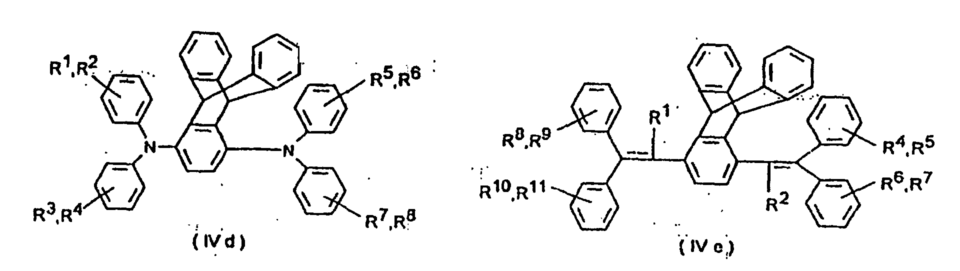 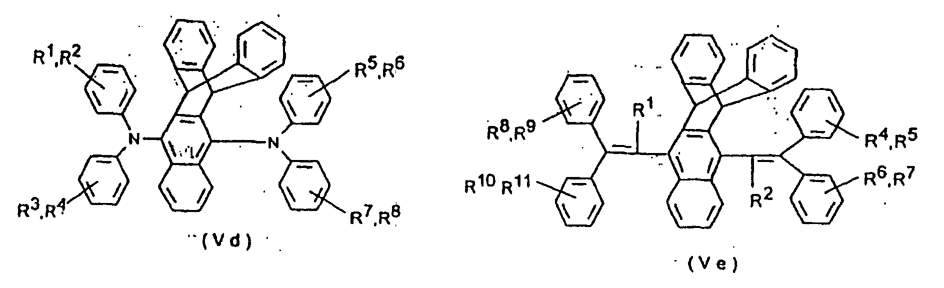 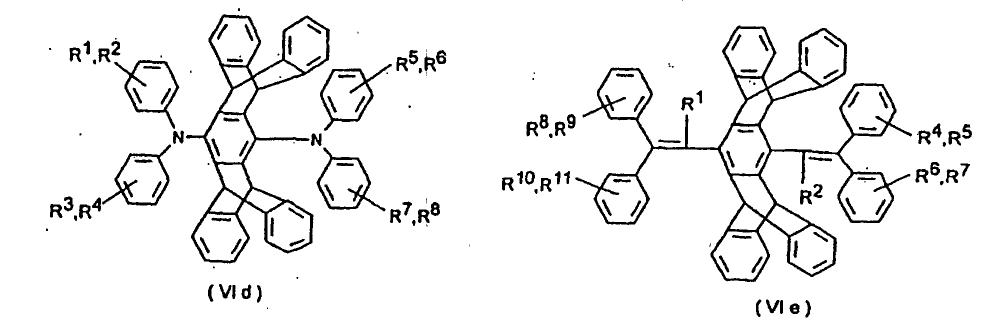 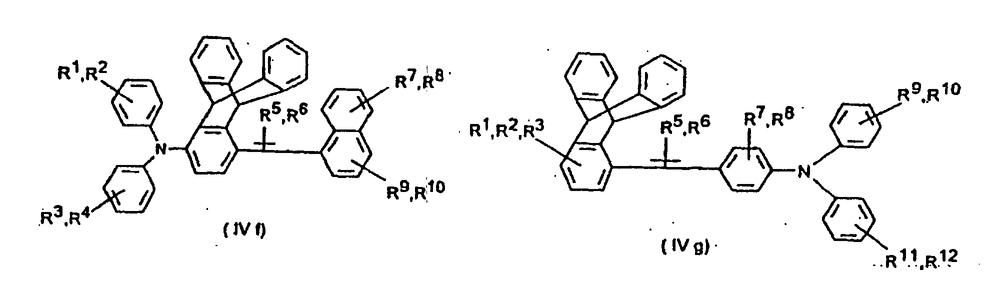 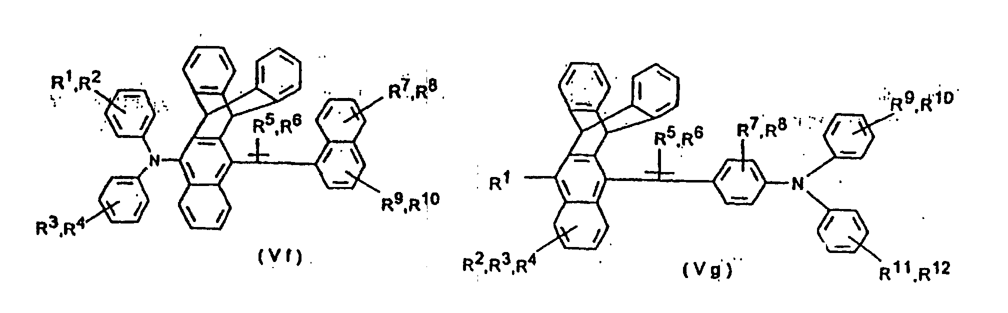 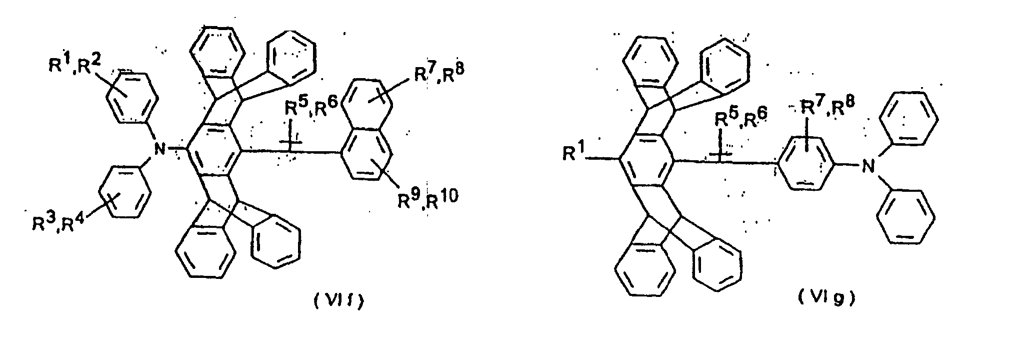 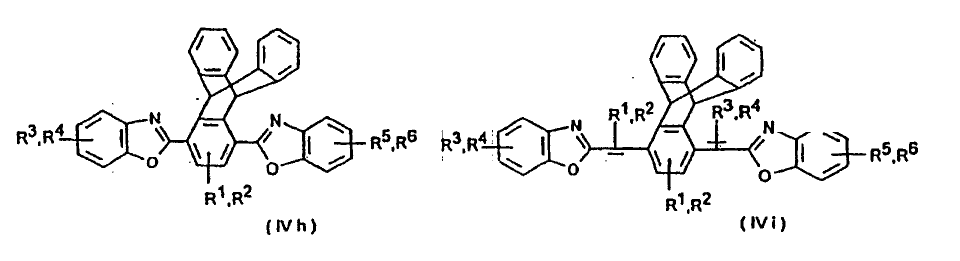 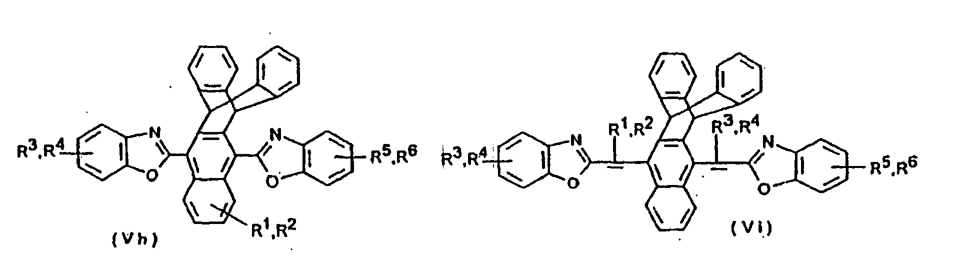 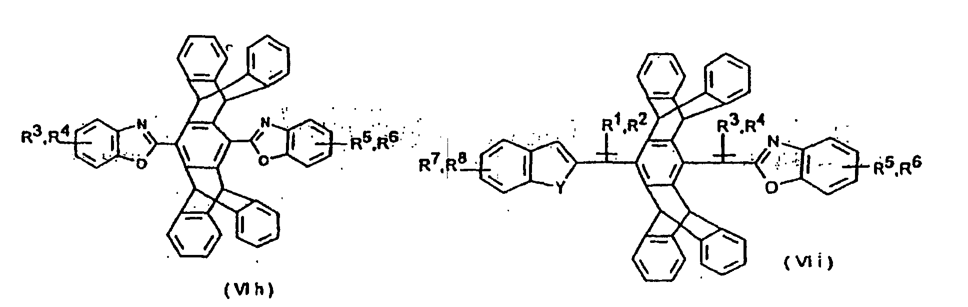 where the symbols have the following meanings:
Y is -O-, -S-, -NR¹¹-, -CR¹⁰R¹¹-;
R¹⁻¹¹ are any one of or a selection from F, Cl, Br, I, CN, NO₂, an unbranched or branched alky group with 1 to 22 C-atoms, where one or more of the -CH₂-groups may be substituted with -O-, -S-, -SO₃-, -O-CO-, -CO-O-, an aryl or heteroaryl group ( with respectively 4 to 10 C-atoms) subject to the requirement that no two oxygen atoms may be directly bonded to one another and where, one, several or all H-atoms may be replaced by F and where two substituents R³ located in the same ring may be linked together so as to form a ring or other anellated ring systems or, furthermore, may be hydrated (possibly only partially) and can carry substituents, subject to the requirement that the number of the substituents is not greater than the total number of C-atoms.

8. Triptycene derivative of the Formula (II) 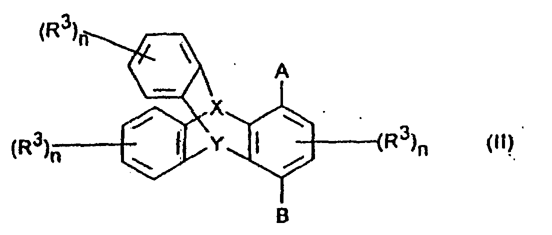 where the symbols and indices have the following meanings:
X, Y, are any one of or a selection from CR¹, N, P, As, SiR²;
R¹ is any one of or a selection from H, halogen, pseudo-halogen or a hydrocarbon residue having 1 to 30 C-atoms, which may also contain heteroatoms,
R² is any one or a selection from hydrocarbon residue(s) with 1 to 30 C-atoms, which may also contain hetero-atoms;
R³ is any one of or a selection from F, Cl, Br, I, CN, NO₂, an unbranched or branched alky group with 1 to 22 C-atoms, where one or more of the -CH₂ groups may be substituted with -O-, -S-, -SO₃-, -O-CO-, -CO-O-, an aryl or heteroaryl group (with respectively 4 to 10 C-atoms) subject to the requirement that no two oxygen atoms may be directly bonded to one another and where one, several or all H-atoms may be replaced by F and where two substituents R³ located in the same ring may be linked together so as to form a ring or other anellated ring systems or, furthermore, may be hydrated (possibly partially) and can carry substituents, subject to the requirement that he number of the substituents is not greater than the total number of C-atoms
n is any one of or a selection from 0, 1, 2, 3 , 4 5;
A, B are any one of or a selection from groups of the formula
(-M)ₐ(-E)_{b}(-M)_{c}-(-E)_{d}(-M)ₑ(-E)_{f}(-M)_{g}(-E)ₕ-R⁴
where the symbols and indices have the following meanings:
M is any one of or a selection from -CR⁵=CR⁶, -C≡C-, -CR⁷=N-, -N=CR⁷-,
E is any one of or a selection from pyrazine-2,5-diyl, pyridazine-3,6-diyl, pyridine-2,5-diyl, pyrimidipe-2,5-diyl, (1,3,4)-thiadiazole-2,5-diyl, 1,3-thiazole-2,4-diyl, 1,3-thiazole-2,5-diyl, thiophene-2,4-diyl, thiophene-2,5-diyl, naphthalene-2,6-dyl, naphthalene-1,4-diyl or naphthalene-1,5-diyl where one or two CH-groups may be replaced by N, 1,3-oxazole-2,4-diyl, 1,3-oxazole-2,5-diyl, (1,3,4)-oxadizole-2,5-diyl, 4, 4'-biphenylene, anthracenediyl, carbazole-diyl, benzoxazole-diyl, indene-2,5-diyl, indene-2,6-diyl, where in the ring systems one or more H-atoms may be substituted with R⁸ residues;
R⁵, R⁶, R⁷ are any one of or a selection from
a) hydrogen, -F, -Cl, -CF₃, -CN, NR⁹R¹⁰
b) a straight-chain or a branched-chain alkyl residue (with or without an asymmetrical C-atom) with 1 to 20 C-atoms, where
b1) one or more non-adjacent and non-terminal CH₂- groups may be replaced with -O-, -S-, -CO-, -CO-O-, -O-CO-, O-CO-O- or Si(CH₃)₂- and / or
b2) one or more CH₂-groups may be replaced by -CH=CH-, -C≡C-, cyclopropane-1,2-diyl, 1,4-phenylene, 1,4-cyclohexylene or 1,3-cyclopentylene and/or
b3) one or more H-atoms may be replaced by F, CN and / or Cl
R⁴ is any one of or a selection from
a) -F, -Cl, -CF₃, -CN, NR⁹NR¹⁰
b) a straight-chain or a branched-chain alkyl residue (with or without an asymmetrical C-atom) with 1 to 20 C-atoms, where
b1) one or more non-adjacent and non-terminal CH₂- groups may be replaced with -O-, -S-, -CO-, -CO-O-, -O-CO-, -O-CO-O- or Si(CH₃)₂- and / or
b2) one or more CH₂-groups may be replaced by -CH=CH-, -C≡C-, cyclopropane-1,2-diyl, 1,4-phenylene, 1,4-cyclohexylene or 1,3-cyclopentylene and/or
b3) one or more H-atoms may be replaced by F, CN and / or Cl
R⁸ is any one of or a selection from
a) -F, -Cl, -CF₃, -CN, NO₂-
b) a straight-chain or a branched-chain alkyl residue (with or without an asymmetrical C-atom) with 1 to 20 C-atoms, where
b1) one or more non-adjacent and non-terminal CH₂- groups may be replaced with -O-, -S-, -CO-, -CO-O-, -O-CO-, -O-CO-O-, -NH-, N( C₁-C₁₀-alkyl), -N-phenyl-, -N-tolyl-, -N(C₂H₅-OCH₃)- or -Si(CH₃)₂- and / or
b2) one or more CH₂-groups may be replaced by -CH=CH-, -C≡C-, 1,4-phenylene and / or
b3) one or more H-atoms may be replaced by F, CN and / or Cl
R⁹, R¹⁰ are any one of or a selection from
a) hydrogen
b) a straight-chain or a branched alkyl residue (with or without an asymmetric C-atom), with 1 to 20 C-atoms, where
b1) one or more CH₂-groups not adjacent to one another or the nitrogen may be replaced by -O-, -S-, -CO-, -CO-O-, -O-CO-, -O-CO-O- or -Si(CH₃)₂ and / or
b2) one or more CH₂ groups may be replaced by -CH=CH-, -C≡C-, cyclopropane-1,2diyl, 1,4-phehylene, 1,4-cyclohexylene or 1,3 cyclopentylene and / or
b3) one or more H-atoms may be replaced by F, CN and / or Cl and
b4) R⁹ and R¹⁰ can together form a ring;
a, b, c, d, e, f, g, and h independently of one another are 0 or 1.

9. Triptycene derivatives represented by Formula III 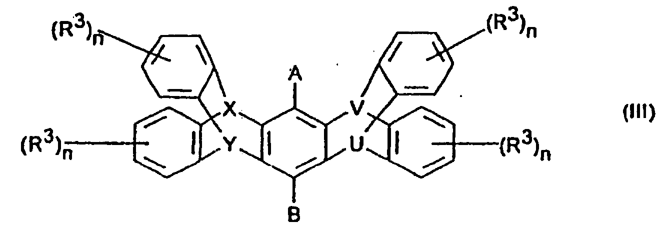 where the symbols and indices have the following significance:
X, Y, U, V are any one of or a selection from CR¹, N, P, As, SiR²;
R¹ is any one of or a selection from H, halogen, pseudo-halogen or a hydrocarbon residue having 1 to 30 C-atoms, which may also contain hetero-atoms
R² is any one or a selection from hydrocarbon residue(s) with 1 to 30 C-atoms, which may also contain hetero-atoms;
R³ is any one of or a selection from F, Cl, Br, I, CN, NO₂, an unbranched or branched alky group with 1 to 22 C-atoms, where one or more of the -CH₂ groups may be substituted with -O-, -S-, -SO₃-, -O-CO-, -CO-O-, an aryl or heteroaryl group (with respectively 4 to 10 C-atoms) subject to the requirement that no two oxygen atoms may be directly bonded to one another and where one, several or all H-atoms may be replaced by F and where two substituents R³ may be linked together so as to form a ring or other anellated ring system or, furthermore, may be hydrated (possibly partially) and can carry substituents, subject to the requirement that the number of the substituents is not greater than the total number of C-atoms;
n is any one of or a selection from 0, 1, 2, 3 , 4, 5;
A, B are any one or a selection from group(s) of the formula
(-M)ₐ(-E)_{b}(-M)_{c}-(-E)_{d}(-M)ₑ(-E)_{f}(-M)_{g} (-E)ₕ-R⁴
where the symbols and indices have the following meanings
M is any one of or a selection from -CR⁵=CR⁶, -C≡C-, -CR⁷=N-, -N=CR⁷-,
E is any one of or a selection from pyrazine-2,5-diyl, pyridazine-3,6-diyl, pyridine-2,5-diyl, pyrimidiae-2,5-diyl, (1,3,4)-thiadiazole-2,5-diyl, 1,3-thiazole-2,4-diyl, 1,3-thiazole-2,5-diyl, thiophene-2,4-diyl, thiophene-2,5-diyl, naphthalene-2,6-diyl, naphthalene-1,4-diyl or naphthalene-1,5-diyl where one or two CH-groups may be replaced by N, 1,3-oxazole-2,4-diyl, 1,3-oxazole-2,5-diyl, (1,3,4)-oxadiazole-2,5-diyl, 4,4'-biphenylene, anthracene-diyl, carbazole-diyl, benzoxazole-diyl, indene-2,5-diyl, indene-2,6-diyl, where in the ring systems one or more H-atoms may be substituted with R⁸ residues;
R⁴, R⁵, R⁶, R7 are any one of or a selection from
a) hydrogen, -F, -Cl, -CF₃, -CN, NR⁹R¹⁰
b) a straight-chain or a branched-chain alkyl residue (with or without an asymmetrical C-atom) with 1 to 20 C-atoms, where
b1) one or more non-adjacent and non-terminal CH₂- groups may be replaced with -O-, -S-, -CO-, -CO-O-,-O-CO-, -O-CO-O-, or Si(CH₃)₂- and / or
b2) one or more CH₂-groups may be replaced by -CH=CH-, C≡C-, cyclopropane-1,2-diyl, 1,4-phenylene, 1,4 cyclohexylene or 1,3-cyclopentylene and / or
b3) one or more H-atoms may be replaced by F, CN and / or Cl
R⁸ is any one of or a selection from
a) -F, -Cl, -CF₃, -CN, NO₂-
b) a straight-chain or a branched-chain alkyl residue (with or without an asymmetrical C-atom) with 1 to 20 C-atoms, where
b1) one or more non-adjacent and non-terminal CH₂- groups may be replaced with -O-, -S-, -CO-, -CO-O-, -O-CO-, -O-CO-O-, -NH-, N(C₁-C₁₀-alkyl), -N-phenyl-, -N-tolyl-, -N(C₂H₅-OCH₃)- or -Si(CH₃)₂- and / or
b2) one or more CH₂-groups may be replaced by -CH=CH-, -C≡C-, 1,4-phenylene and / or
b3) one or more H-atoms may be replaced by F, CN and / or Cl
R⁹, R¹⁰ are any one of or a selection from
a) hydrogen
b) a straight-chain or a branched alkyl residue (with or without an asymmetric C-atom), with 1 to 20 C-atoms, where
b1) one or more CH₂-groups not adjacent to one another or the nitrogen may be replaced by -O-, -S-, -CO-, -CO-O-, -O-CO-, -O-CO-O- or -Si(CH₃)₂- and / or
b2) one or more CH₂-groups may be replaced by -CH=CH-, -C≡C-, cyclopropane-1,2-diyl, 1,4-phenylene, 1,4-cyclohexylene or 1,3-cyclopentylene and / or
b3) one or more H-atoms may be replaced by F, CN and / or Cl and
b4) R⁸ and R⁹ can together form a ring;
a, b, c, d, e, f, g, and h independently of one another are 0 or 1.

10. An electroluminescent device containing one or more active layers, **characterised in that** at least one active layer contains one or more triptycene derivatives in accordance with one or more of the claims 1 to 6.

## Revendications

1. Utilisation de dérivés de la triptycène de formule (I) dans des dispositifs électroluminescents 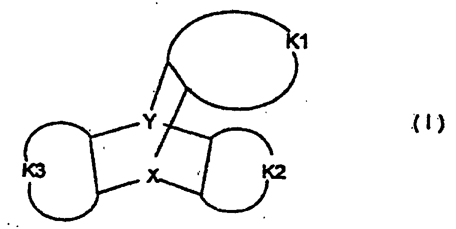 où les symboles à la formule possèdent les significations suivantes :
K¹, K², K³ identiques ou différents représentent des systèmes mono- ou polycycliques, qui présentent éventuellement des hétéroatomes et qui sont éventuellement substitués ;
X, Y identiques ou différents représentent un groupe CR¹, N, P, As, SiR² ;
R¹ identiques ou différents représentent un atome d'hydrogène, un atome d'halogène, un groupe pseudohalogène ou un reste hydrocarboné présentant 1 à 30 atomes de carbone, qui présente éventuellement des hétéroatomes également ;
R² identiques ou différents représentent un reste hydrocarboné présentant 1 à 30 atomes de carbone, qui présente éventuellement des hétéroatomes également.

2. Utilisation selon la revendication 1, **caractérisée en ce qu'**au moins un des groupes K¹⁻³ à la formule (I) est un fluorophore.

3. Utilisation selon la revendication 1, **caractérisée en ce que** les groupes K¹⁻³ sont des systèmes conjugués.

4. Utilisation selon une ou plusieurs des revendications 1 à 3, **caractérisée en ce que** le dérivé dé la triptycène est un composé de formule (II) ou de formule (III), 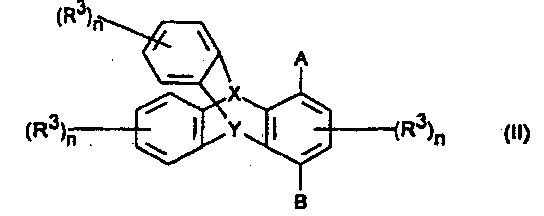 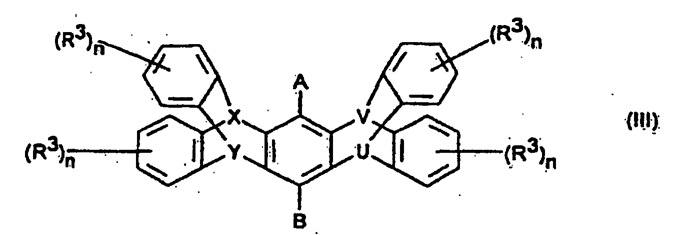 les symboles et les indices possèdent les significations suivantes :
X, Y, U, V identiques ou différents représentent un groupe CR¹, N, P, As, SiR² ;
R¹ identiques ou différents représentent un atome d'hydrogène, un atome d'halogène, un groupe pseudohalogène ou un reste hydrocarboné présentant 1 à 30 atomes de carbone, qui présente éventuellement des hétéroatomes également ;
R² identiques ou différents représentent un reste hydrocarboné présentant 1 à 30 atomes de carbone, qui présente éventuellement des hétéroatomes également ;
R³ identiques ou différents représentent un atome de F, de Cl, de Br, d'I, un groupe CN, NO₂, un groupe alkyle linéaire ou ramifié présentant 1 à 22 atomes de carbone, un ou plusieurs groupes -CH₂- peuvent être remplacés par -O-, -S-, -SO₃-, -O-CO-, -CO-O-, aryle ou hétéroaryle (chacun présentant 4 à 10 atomes de carbone), à condition que deux atomes d'oxygène ne puissent pas être liés directement l'un à l'autre, et un, plusieurs ou tous les atomes d'hydrogène peuvent être remplacés par des atomes de fluor, et deux substituants R³ qui se trouvent sur le même cycle peuvent être reliés l'un à l'autre en formant un cycle ou un autre système cyclique condensé, ou aussi peuvent être, éventuellement partiellement, hydrogénés et porter des substituants à condition que le nombre des substituants ne soit pas supérieur au nombre total des atomes de carbone ;
n identiques ou différent valent .0, 1, 2, 3, 4, 5 ;
A, B identiques ou différents représentent des groupes de formule
(-M)ₐ(-E)_{b}(-M)_{c}-(-E)_{d}(-M)ₑ(-E)_{f}(-M)_{g}(-E)ₕ-R⁴
où les symboles et les indices possèdent les significations suivantes :
M identiques ou différents représentent -CR⁵=CR⁶, -C≡C-, -CR⁷=N-, -N=CR⁷- ;
E identiques ou différents représentent un groupe pyrazine-2,5-diyle, pyridazine-3,6-diyle, pyridine-2,5-diyle, pyrimidine-2,5-diyle, (1,3,4)-thiadiazole-2,5-diyle, 1,3-thiazole-2,4-diyle, 1,3-thiazole-2,5-diyle, thiophène-2,4-diyle, thiophène-2,5-diyle, naphtalène-2,6-diyle, naphtalène-1,4-diyle ou naphtalène-1,5-diyle, un ou deux groupes CH pouvant être remplacés par un atome de N, 1,3-oxazole-2,4-diyle, 1,3-oxazole-2,5-diyle, (1,3,4)-oxadiazole-2,5-diyle, 4,4'-biphénylène, anthracène-diyle, carbazole-diyle, benzoxazole-diyle, indène-2,5-diyle, indène-2,6-diyle, un ou plusieurs atomes d'hydrogène dans les systèmes cycliques peuvent aussi être substitués par des restes R⁸ ;
R⁴, R⁵, R⁶, R⁷ identiques ou différents représentent
a) un atome d'hydrogène, -F, -Cl, -CF₃, -CN, NR⁹R¹⁰,
b) un reste alkyle linéaire ou ramifié (avec ou sans atome de carbone asymétrique) présentant 1 à 20 atomes de carbone,
b1) un ou plusieurs groupes CH₂ non adjacents et non terminaux peuvent être remplacés par -O-, -S-, -CO-, -CO-O-, -O-CO-, -O-CO-O- ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène peuvent être remplacés par F, CN et/ou Cl et
R⁸ identiques ou différents représentent
a) -F, -Cl, -CF₃, -CN, NO₂-,
b) un reste alkyle linéaire ou ramifié (avec ou sans atome de carbone asymétrique) présentant 1 à 20 atomes de carbone,
b1) un ou plusieurs groupes CH₂ non adjacents et non terminaux peuvent être remplacés par -O-, -S-, -CO-, -CO-O-, -O-CO-, -O-CO-O-, -NH-, -N-alkyle en C₁-C₁₀, -N-phényl-, -N-tolyl-, -N(C₂H₅-OCH₃)- ou -Si(CH₃)₂- et/ou
b2) un ou plusieurs groupes CH₂ peuvent être remplacés par un groupe -CH=CH-, -C≡C-, 1,4-phénylène et/ou
b3) un ou plusieurs atomes d'hydroègne peuvent être remplacés par F, CN et/ou Cl ;
R⁹, R¹⁰ identiques ou différents représentent
a) un atome d'hydrogène,
b) un reste alkyle linéaire ou ramifié (avec ou sans atome de carbone asymétrique) présentant 1 à 20 atomes de carbone,
b1) un ou plusieurs groupes CH₂ entre eux ou adjacents à l'atome d'azote ne peuvent être remplacés par -O-, -S-, -CO-, -CO-O-, -O-CO-, -O-CO-O- ou -Si(CH₃)₂- et/ou
b2) un ou plusieurs groupes CH₂ peuvent être remplacés par un groupe -CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène peuvent être remplacés par F, CN et/Cl et
b4) R⁸ et R⁹ ensemble peuvent aussi former un cycle ;
a, b, c, d, e, f, g, h indépendamment les uns des autres valent 0 ou 1.

5. Utilisation selon la revendication 4, **caractérisée en ce que** la somme des indices a-h à la formule (II) ou (III) s'élève à au moins 1.

6. Utilisation selon la revendication 5, **caractérisée en ce qu'**on utilise un dérivé de la triptycène de formule (IV), (V) ou (VI) 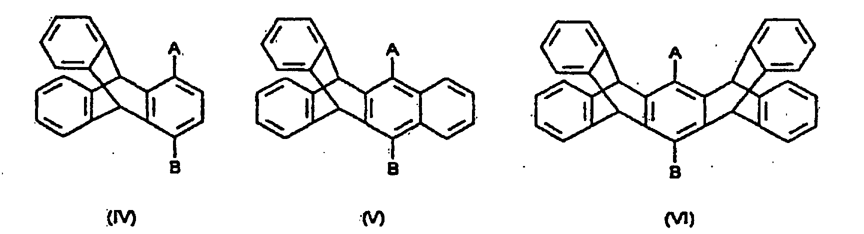 les groupes A et B possèdent les mêmes significations qu'aux formules (II), (III) à la revendication 4.

7. Utilisation selon la revendication 6, **caractérisée par** un dérivé de la triptycène pris dans le groupe (IV), (V), (VI) a-i : 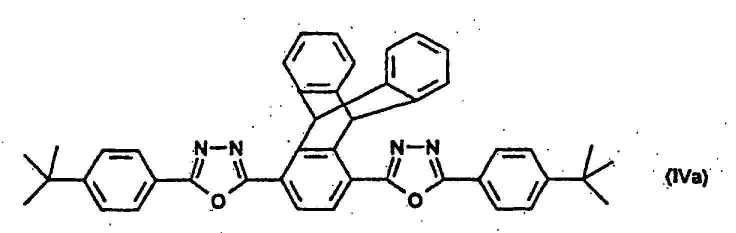 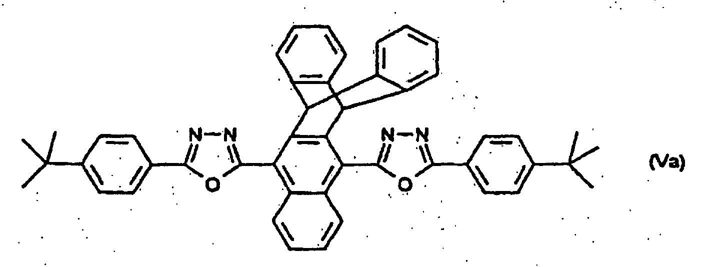 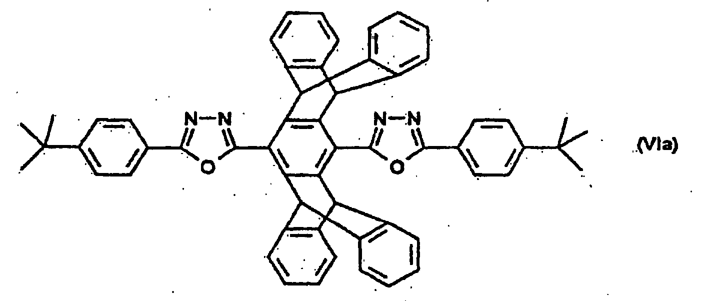 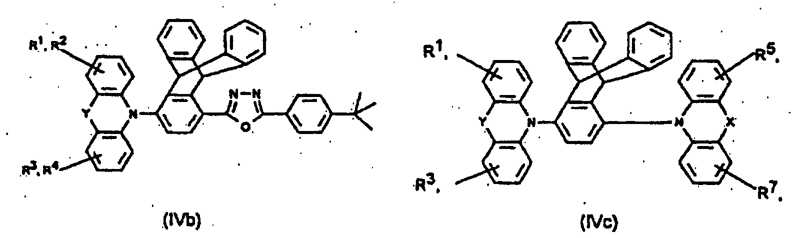 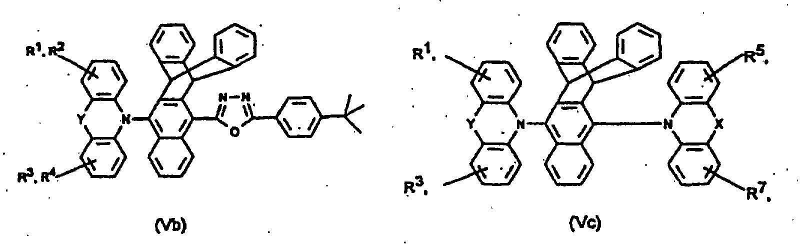 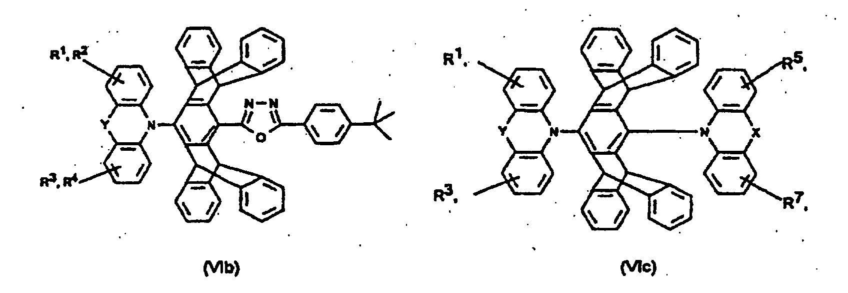 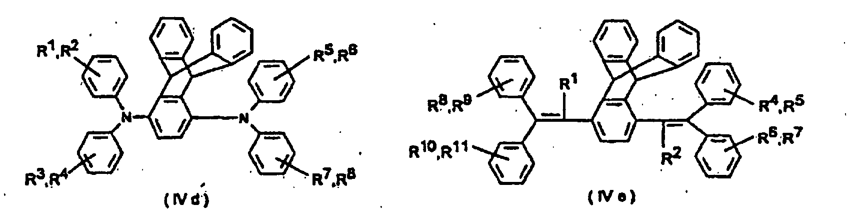 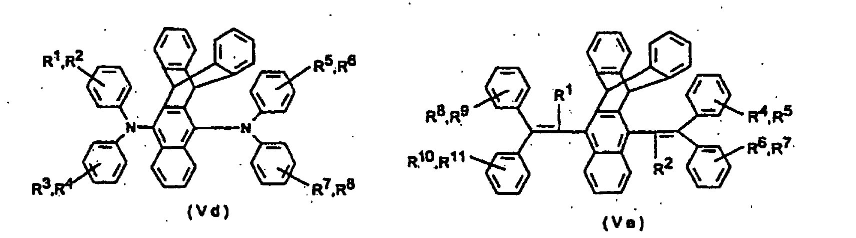 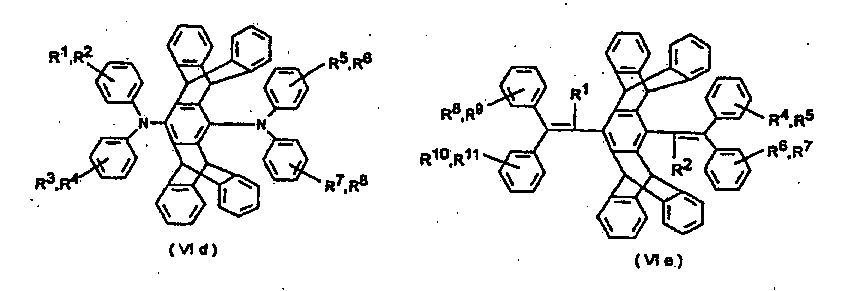 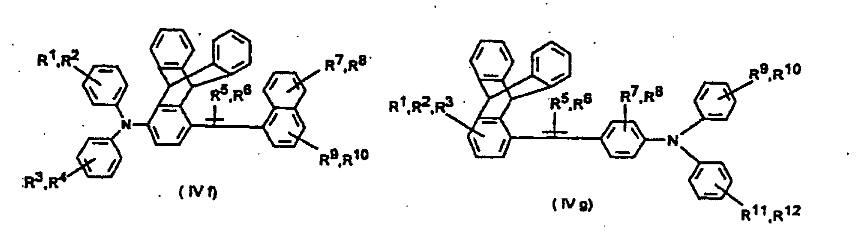 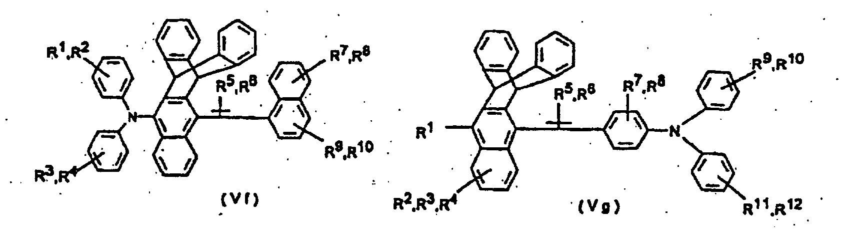 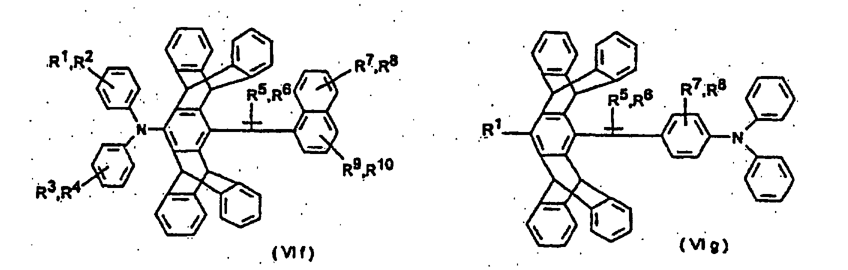 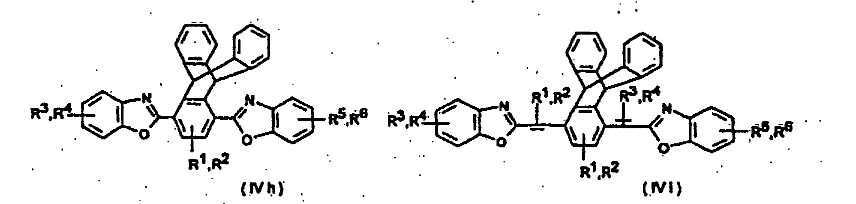 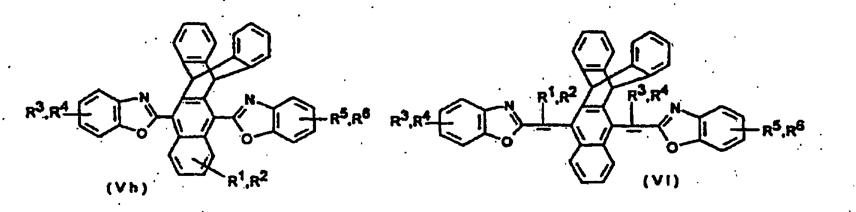 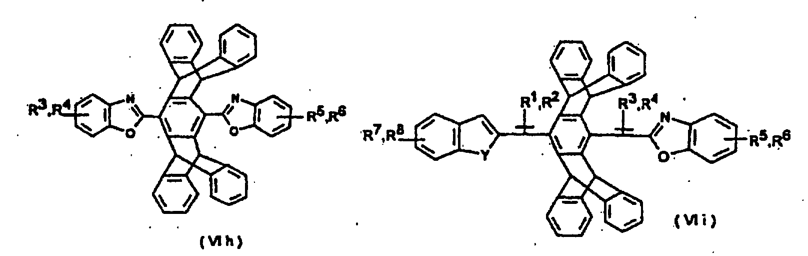 où les symboles possèdent les significations suivantes :
Y représente -O-, -S-, -NR¹¹-, -CR¹⁰R¹¹ ;
R¹⁻¹¹ identiques ou différents représentent F, Cl, I, CN, NO₂, un groupe alkyle linéaire ou ramifié présentant 1 à 22 atomes de carbone, un ou plusieurs groupes CH₂ peuvent être remplacés par -O-, -S-, -SO₃-, -O-CO-, -CO-O-, aryle ou hétéroaryle (chacun avec 4 à 10 atomes de carbone), à condition que deux atomes d'oxygène adjacents ne puissent pas être liés l'un à l'autre, et un, plusieurs ou tous les atomes d'hydrogène peuvent être remplacés par F, et deux substituants R² se trouvant sur le même cycle peuvent être reliés l'un à l'autre en formant un cycle ou un autre système cyclique condensé, ou aussi peuvent être, éventuellement partiellement, hydrogénés et porter des substituants, à condition que le nombre des substituants ne soit pas supérieur au nombres des atomes de carbone.

8. Dérivé de la triptycène de formule (II) 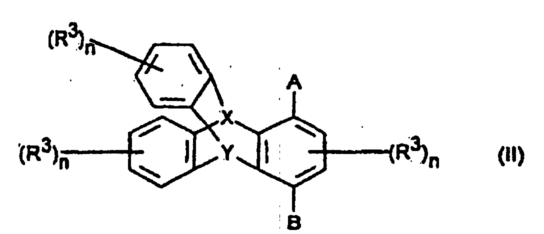 où les symboles et les indices possèdent les significations suivantes :
X, Y identiques ou différents représentent un groupe CR¹, N, P, As, SiR² ;
R¹ identiques ou différents représentent un atome d'hydrogène, un atome d'halogène, un groupe pseudohalogène ou un reste hydrocarboné présentant 1 à 30 atomes de carbone, qui présente éventuellement des hétéroatomes également ;
R² identiques ou différents représentent un reste hydrocarboné présentant 1 à 30 atomes de carbone, qui présente éventuellement des hétéroatomes également ;
R³ identiques ou différents représentent un atome de F, de Cl, de Br, d'I, un groupe CN, NO₂, un groupe alkyle linéaire ou ramifié présentant 1 à 22 atomes de carbone, un ou plusieurs groupes -CH₂- peuvent être remplacés par -O-, -S-, -SO₃-, -O-CO-, -CO-O-, aryle ou hétéroaryle .(chacun présentant 4 à 10 atomes de carbone), à condition que deux atomes d'oxygène ne puissent pas être directement liés l'un à l'autre, et un, plusieurs ou tous les atomes d'hydrogène peuvent être remplacés par des atomes de fluor, et deux substituants R³ qui se trouvent sur le même cycle peuvent être reliés l'un à l'autre en formant un cycle ou un autre système cyclique condensé, ou aussi peuvent être, éventuellement partiellement, hydrogénés et porter des substituants, à condition que le nombre des substituants ne soit pas supérieur au nombre total des atomes de carbone ;
n identiques ou différents valent 0, 1, 2, 3, 4, 5 ;
A, B identiques ou différents représentent des groupes de formule
(-M)ₐ(-E)_{b}(-M)_{c}-(-E)_{d}(-M)ₑ(-E)_{f}(-M)_{g}(-E)ₕ-R⁴
où les symboles et indices possèdent les significations suivantes :
M identiques ou différents représentent -CR⁵=CR⁶, -C≡C-, -CR⁷=N-, -N=CR⁷- ;
E identiques ou différents représentent un groupe pyrazine-2,5-diyle, pyridazine-3,6-diyle, pyridine-2,5-diyle, pyrimidine-2,5-diyle, (1,3,4)-thiadiazol-2,5-diyle, 1,3-thiazole-2,4-diyle, 1,3-thiazole-2,5-diyle, thiophène-2,4-diyle, thiophène-2,5-diyle, naphtalène-2,6-diyle, naphtalène-1,4-diyle ou naphtalène-1,5-diyle, un ou plusieurs groupes CH pouvant être remplacés par N, 1,3-oxazole-2,4-diyle, 1,3-oxazole-2,5-diyle, (1,3,4)-oxadiazole-2,5-diyle, 4,4'-biphénylène, anthracène-diyle, carbazole-diyle, benzoxazole-diyle, indène-2,5-diyle, indène-2,6-diyle, un ou plusieurs atomes d'hydrogène dans les systèmes cycliques pouvant être substitué par des restes R⁸ ;
R⁵, R⁶, R⁷ identiques ou différents représentent
a) un atome d'hydrogène, -F, -Cl, -CF₃, -CN, NR⁹R¹⁰,
b) un reste alkyle linéaire ou ramifié (avec ou sans atome de carbone asymétrique) présentant 1 à 20 atomes de carbone,
b1) un ou plusieurs groupes CH₂ non adjacents et non terminaux peuvent être remplacés par -O-, -S-, -CO-, -CO-O-, -O-CO-, -O-CO-O- ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène peuvent être remplacés par F, CN et/ou Cl ;
R⁴ identiques ou différents représentent
a) -F, -1, -CF₃, -CN, NR⁹R¹⁰,
b) un reste alkyle linéaire ou ramifié (avec ou sans atome de carbone asymétrique) présentant 1 à 20 atomes de carbone
b1) un ou plusieurs groupes CH₂ non adjacents et non terminaux peuvent être remplacés par -O-, -S-, -CO-, -CO-O-, -O-CO-, -O-CO-O- ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène peuvent être remplacés par F, CN et/ou Cl et
R⁸ identiques ou différents représentent
a) -F, -Cl, -CF₃, -CN, NO₂-,
b) un reste alkyle linéaire ou ramifié (avec ou sans atome de carbone asymétrique) présentant 1 à 20 atomes de carbone,
b1) un ou plusieurs groupes CH₂ non adjacents et non terminaux peuvent être remplacés par -O-, -S-, -CO-, -CO-O-, -O-CO-, -O-CO-O-, -NH-, -N-alkyle en C₁-C₁₀, -N-phényl-, -N-tolyl-, -N(C₂H₅-OCH₃)- ou -Si(CH₃)₂- .et/ou
b2) un ou plusieurs groupes CH₂ peuvent être remplacés par un groupe -CH=CH-, -C≡C-, 1,4-phénylène et/ou
b3) un ou plusieurs atomes d'hydrogène peuvent être remplacés par F, CN et/ou Cl ;
R⁹, R¹⁰ identiques ou différents représentent
a) un atome d'hydrogène,
b) un reste alkyle linéaire ou ramifié (avec ou sans atome de carbone asymétrique) présentant 1 à 20 atomes de carbone où
b1) un ou plusieurs groupes CH₂ entre eux ou adjacents à l'atome d'azote ne peuvent pas être remplacés par -O-, -S-, -CO-, -CO-O-, -O-CO-, -O-CO-O- ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène peuvent être remplacés par F, CN et/ou Cl et
b4) R⁸ et R⁹ conjointement peuvent aussi former un cycle,
a, b, c, d, e, f, g, h indépendamment les uns des autres valent 0 ou 1.

9. Dérivés de la triptycène de formule III. 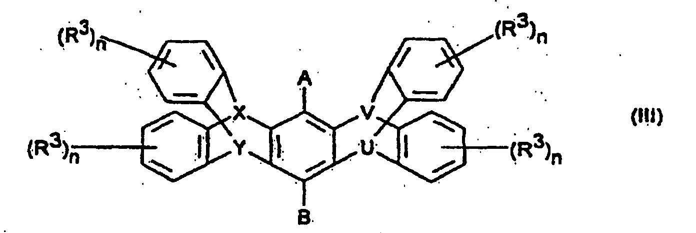 où les symboles et les indices possèdent les significations suivantes :
X, Y, U, V identiques ou différents représentent un groupe CR¹, N, P, As, SiR² ;
R¹ identiques ou différents représentent un atome d'hydrogène, un atome, d' halogène, un groupe pseudohalogène ou un reste hydrocarboné présentant 1 à 30 atomes de carbone, qui présente éventuellement des hétéroatomes,
R² identiques ou différents représentent reste hydrocarboné présentant 1 à 30 atomes de carbone, qui présente éventuellement des hétéroatomes également ;
R³ identiques ou différents représentent un atome de F, de Cl, de Br, d'I, un groupe CN, NO₂, un groupe alkyle linéaire ou ramifié présentant 1 à 22 atomes de carbone, un ou plusieurs groupes -CH₂- peuvent être remplacés par -O-, -S-, -SO₃-, -O-CO-, -CO-O-, aryle ou hétéroaryle (chacun présentant 4 à 10 atomes de carbone) , à condition que deux atomes d'oxygène ne puissent pas être liés directement l'un à l'autre, et un, plusieurs ou tous les atomes d'hydrogène peuvent être remplacés par du fluor, et deux substituants R² qui se trouvent sur le même cycle peuvent être reliés l'un à l'autre en formant un cycle ou un autre système cyclique condensé, ou aussi peuvent être, éventuellement partiellement, hydrogénés et porter des substituants, à condition que le nombre des substituants ne soit pas supérieur au nombre total des atomes de carbone ;
n identiques ou différents valent 0, 1, 2, 3, 4, 5 ;
A, B identiques ou différents représentent des groupes de formule
(-M)ₐ(-E)_{b}(-M)_{c}-(-E)_{d}(-M)ₑ(-E)_{f}(-M)_{g}(-E)ₕ-R⁴
les symboles et indices possèdent les significations suivantes :
M identiques ou différents représentent un groupe -CR⁵=CR⁶, -C≡C-, -CR⁷=N-, -N=CR⁷- ;
E identiques ou différents représentent un groupe pyrazine-2,5-diyle, pyridazine-3,6-diyle, pyridine-2,5-diyle, pyrimidine-2,5-diyle, (1,3,4)-thiadiazole-2,5-diyle, 1,3-thiazole-2,4-diyle, 1,3-thiazole-2,5-diyle, thiophène-2,4-diyle, thiophène-2,5-diyle, naphtalène-2,6-diyle, naphtalène-1,4-diyle ou naphtalène-1,5-diyle, un ou plusieurs groupes CH pouvant être remplacés par N, 1,3-oxazole-2,4-diyle, 1,3-oxazole-2,5-diyle, (1,3,4)-oxadiazole-2,5-diyle, 4,4'-biphénylène, anthracène-diyle, carbazole-diyle, benzoxazole-diyle, indène-2,5-diyle, indène-2,6-diyle, dans les systèmes cycliques, un ou plusieurs atomes d'hydrogène pouvant être substitué par des restes R⁸ ;
R⁴, R⁵, R⁶, R⁷ sont identiques ou différents et représentent
a) un atome d'hydrogène, -F, -Cl, -CF₃, -CN, NR⁹R¹⁰,
b) représente un reste alkyle linéaire ou ramifié (avec ou sans atome de carbone asymétrique) présentant 1 à 20 atomes de carbone,
b1) un ou plusieurs groupes CH₂ non adjacents et non terminaux peuvent être remplacés par -O-, -S-, -CO-, -CO-O-, -O-CO-, -O-CO-O- ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène peuvent être remplacés par F, CN et/ou Cl et
R⁸ identiques ou différents
a) -F, -Cl, -CF₃, -CN, NO₂-,
b) un reste alkyle linéaire ou ramifié (avec ou sans atome de carbone asymétrique) présentant 1 à 20 atomes de carbone
b1) un ou plusieurs groupes CH₂ non adjacents et non terminaux peuvent être remplacés par -O-, -S-, -CO-, -CO-O-, -O-CO-, -O-CO-O-, -NH-, -N-alkyle C₁-C₁₀, -N-phényl-, -N-tolyl-, -N(C₆H₅-OCH₃)- ou -Si(CH₃)₂- et/ou
b2) un ou plusieurs groupes CH₂ peuvent être remplacés par des groupes -CH=CH-, -C≡C-, 1,4-phénylène et/ou
b3) un ou plusieurs atomes d'hydrogène peuvent être remplacés par F, CN et/ou Cl et
R⁹, R¹⁰ identiques ou différents représentent
a) un atome d'hydrogène,
b) un reste alkyle linéaire ou ramifié (avec ou sans atome de carbone asymétrique) présentant 1 à 20 atomes de carbone
b1) un ou plusieurs groupes CH₂ entre eux ou adjacents à l'atome d'azote ne peuvent pas être remplacés par -O-, -S-, -CO-, -CO-O-, -O-CO-, -O-CO-O- ou -Si(CH₃)₂- et/ou
b2) un ou plusieurs groupes CH₂ peuvent être remplacés par des groupes -CH=CH-, -C≡C-, cyclopropane-1,2-diyle, 1,4-phénylène, 1,4-cyclohexylène ou 1,3-cyclopentylène et/ou
b3) un ou plusieurs atomes d'hydrogène peuvent être remplacés par F, CN et/ou Cl et
b4) R⁸ et R⁹ peuvent aussi conjointement former un cycle,
a, b, c, d, e, f, g, h indépendamment les uns des autres valent 0 ou 1.

10. Dispositif électroluminescent, présentant une ou plusieurs couches actives, **caractérisé en ce qu'**au moins une couche active renferme un ou plusieurs dérivés de la triptycène selon une ou plusieurs revendications 1 à 6.
